# EUROPEAN PATENT SPECIFICATION

(11) **EP 3 525 834 B1**
(45) Date of publication and mention of the grant of the patent: **14.04.2021**
(21) Application number: 16784749.0
(22) Date of filing: 13.10.2016
(51) Int. Cl.: A61L 27/26, A61L 27/52, A61K 8/73, A61Q 19/08, A61K 8/04

(54) **COACERVATE HYALURONAN HYDROGELS FOR DERMAL FILLER APPLICATIONS**
KOAZERVAT-HYALURONAN-HYDROGELE FÜR DERMALE FÜLLSTOFFANWENDUNGEN
HYDROGELS D'HYALURONANE COACERVATS POUR DES APPLICATIONS DE COMBLEMENT DERMIQUE

(43) Date of publication of application: 21.08.2019
(73) Proprietor: Allergan, Inc., Irvine, California 92612 (US)
(72) Inventor: LIU, Futian, Lake Forest, California 92630 (US); YU, Xiaojie, Orange, California 92867 (US); VAN EPPS, Dennis E., Goleta, California 93117 (US); STREHIN, Iossif A., Irvine, California 92603 (US)
(74) Representative: Hoffmann Eitle
(86) International application number: PCT/US2016/056897
(87) International publication number: WO 2018/071033

(56) References cited:
- EP-A1- 1 595 534
- US-A1- 2011 229 574

## Description

### BACKGROUND

The present disclosure generally relates to injectable dermal fillers. The dermal fillers are hydrogel compositions comprising an anionic polysaccharide and a cationic polysacharide. More specifically, the hydrogel comprises an ionic complex between a hyaluronic acid and a cationic polysaccharide.

Injectable dermal fillers are gels that act as volumizers in skin, or space occupying agents which fill in the voids within or under the skin to reduce the appearance of wrinkles or other skin defects. Dermal fillers may also be used for sculpting particular soft tissue features, including facial features, or to replace dermal tissue. The dermal filler materials are biologically inert, achieving their goal solely by mechanical pressure against the adjacent tissue. Dermal fillers have been shown to persist in the body for up to 18 months. In order to achieve the desirable results for correcting deep wrinkle or skin defects, or for scuplting particular facial features, it is desireable for these gels to have sufficient lifting capacity, good moldability and/or injectability.

Hyaluronic acid (HA), also known as hyaluronan, is a non-sulfated glycosaminoglycan found in many tissues throughout the human body, including connective, epithelial, and neural tissues. HA is abundant in the different layers of the skin, where it has multiple functions, such as ensuring good hydration, assisting in the organization of the extracellular matrix, acting as a filler material, and participating in tissue repair mechanisms. However, the quantities of HA and other matrix polymers present in the skin, such as collagen and elastin, decrease with age. For example, repeated exposed to ultraviolet light from the sun or other sources causes dermal cells to both decrease their production of HA as well as increase the rate of its degradation. This loss of materials results in various skin conditions such as wrinkling, hollowness, loss of moisture and other undesirable conditions that contribute to the appearance of aging.

Injectable dermal fillers have been successfully used in treating the aging skin, and for reducing other skin defects, such as scars or soft tissue contour defects. The fillers can replace lost endogenous matrix polymers, or enhance/facilitate the function of existing matrix polymers, in order to treat these skin conditions.

Due to its excellent biocompatibility, HA has been considered an ideal candidate for dermal filler applications. HA is composed of repeating disaccharide units bearing free carboxylate groups; thus, HA is an anionic polysaccharide at physiological pH.

In order to be effective in optimal duration as a dermal filler, HA is usually chemically crosslinked, since non-crosslinked HA has a short persistance time *in vivo.* Chemical crosslinking methods include Michael addition, thiol-ene coupling, free radical polymerization, carbodiimide chemistry (e.g., 1-ethyl-3-(3-dimethylaminopropyl) carbodiimide (EDC)) using a di- or polyamine as a crosslinker, and epoxy chemistry using 1,4-butanediol diglycidyl ether (BDDE) as a crosslinker. Other commonly employed chemical crosslinkers include divinyl sulfone (DVS) and 1,2,7,8-diepoxyoctane (DEO), and further agents disclosed herein. These chemical crosslinking methods provide HA with a covalently bonded framework.

One limitation of conventional chemically crosslinked hydrogels is that they often require tedious purification steps. Another limitation of chemically crosslinked HA hydrogels made using conventional crosslinking processes is that they are generally not injectable from the moment they are crosslinked, and must be further processed to make them injectable as dermal fillers. For example, in order to be injectable through a fine needle, crosslinked HA gels are typically re-hydrated to a desired concentration and then further processed by either sizing the hydrated gel through a fine porous screen or a homogenization process. A non-crosslinked HA is sometimes added as yet a further processing step in order to enhance lubricity and injectability of the gel. One drawback of the further processing of the crosslinked HA hydrogel is that the gel normally loses its cohesivity during these additional processing steps, especially in the case of a hydrogel with a high storage modulus (G'). Therefore, lifting capacity and moldability of the materials may be compromised for use in treating the skin or soft tissue, for example, the materials may be compromised for deep wrinkle and sculpting applications.

Another approach to overcoming the limitations of chemically crosslinked hydrogels is to inject low viscosity HA containing chemical- or UV-crosslinkable functional groups, and to form the hydrogel *in situ.* Drawbacks to this approach are that the precursors are reactive, difficult to prepare, handle and store, and suffer from low doctor usability

EP 1 595 534 A1 discloses an hydrogel composition comprising a cationic modified dextran and an opt. cross-linked anionic polymeric material e.g. HA and opt. an active compound. US 2011/229574 A1 discloses injectable soft tissue fillers including cross-linked HA, one or more biocompatible polymers e.g. chitosan (see [0031]) and an active agent (antioxidant..) in a physiologically acceptable carrier (PBS).

There remains a need for better dermal fillers for treating and improving the appearance of soft tissues, including the skin.

### SUMMARY

The scope of protection is defined by the claims. Any references in the description to methods of treatments refer either to non-therapeutic methods or to the products of the present invention for use in a method for treatment of the human or animal body by therapy. Any "embodiment" or " example" which is disclosed in the description but is not covered by the claims should be considered as presented for illustrative purpose only.

The present invention provides a dermal filler composition comprising a coacervate hydrogel that is useful for treating a soft tissue of a subject, such as the skin. The coecervate hydrogel comprises a noncovalent complex based on charge-charge interactions between an "anionic HA" polysaccharide, hyaluronic acid, and a cationic polysaccharide. The complexes arise through electrostatic and/or ionic interactions between the anions and cations of the polysaccharides. More specifically, the interactions occur between the anionic HA polysaccharide ions and the cationic polysaccharide ions. The interactions arise through ionic interactions between the carboxylate anions of HA and the cations of the cationic polysaccharides. Consequently, the binding interactions between polysaccharides of the coacervate HA hydrogels of the invention are more dynamic than the fixed interactions of traditional crosslinked HA hydrogels, which are joined by covalent bonds. For example, the anionic-cationic interactions of the present coacervate HA hydrogels can be disrupted, *e.g.,* under shearing or other conditions, and the same or different anionic-cationic interactions can be formed between the same or different anion-cation pairs. In this way, the coacervate hydrogels are "self-healing," and advantageously remain cohesive and moldable without the need for numerous homegenization or sizing steps prior to use. Thus, the coacervate HA-based hydrogels of the present invention provide numerous advantages over conventional, chemically crosslinked HA-based dermal fillers. For example, many of the hydrogels of the present invention can be processed as dermal fillers without the need for some of the tedious chemical crosslinking and complex purification steps that are sometimes necessary to remove chemical residues from chemically crosslinked gels, without the need for further processing by sizing, homogenizing, which can disrupt gel integrity and result in the formation of gel particles that are not conducive to injectability, or without adding non-crosslinked HA to enhance lubricity or injectability.

The coacervate HA-based hydrogels of the invention have sufficient or improved properties, including sufficient or improved cohesivity, moldability, lifting capacity and/or injectability for their desired application as dermal filler materials relative to chemically crosslinked HA-based hydrogels.

There is provided a dermal filler generally comprising a coacervate HA hydrogel. The hydrogel comprises an anionic HA and a cationic polysaccharide. In some aspects, the hydrogel comprises an ionic complex between an anionic HA and a cationic polysaccharide.

In some aspects, the hydrogel comprises an anionic HA polysaccharide, which is hyaluronic acid (HA) itself. In other aspects, the anionic HA is a "modifed HA", i.e., an HA that has been modified to introduce one or more anionic groups other than carboxylate, and wherein the anionic groups may be the same or different, as further defined herein. In some aspects, the hydrogel comprises an anionic HA which is homoanionic. In other aspects, the hydrogel comprises an anionic HA which is heteroanionic. In some aspects, the hydrogel comprises an anionic HA selected from a non-crosslinked anionic HA, a crosslinked anionic HA, and a mixture thereof. In some aspects, the hydrogel comprises non-crosslinked HA, crosslinked HA, or a mixture thereof.

The coacervate hydrogel comprises a cationic polysaccharide. In some aspects, the cationic polysaccharide has been modified to introduce one or more additional cationic groups, and/or different cationic groups, relative to its unmodified form. In some aspects, the hydrogel comprises an unmodified or modified cationic polysaccharide which is homocationic. In other aspects, the hydrogel comprises an unmodified or modified cationic polysaccharide which is heterocationic. In some aspects, the cationic polysaccharide is chitosan. In other aspects, the cationic polysaccharide is trimethyl chitosan.

In other aspects of the invention, there is provided a cationic polysaccharide which is a "cationic HA," and methods of preparing the same. In further aspects of the invention, there is provided a coacervate hydrogel comprising the cationic HA. In yet further aspects, the cationic HA is selected from a non-crosslinked cationic HA, a crosslinked cationic HA, and a mixture thereof.

In another aspect, there is provided dermal filler compositions further comprising cosmetic agents or other agents such as vitamins, antioxidants and/or skin lightening agents.

In another aspect, the coacervate HA hydrogels of the invention are provided in a physiologically acceptable carrier. In some aspects, the physiologically acceptable carrier is phosphate buffered saline or non-crosslinked HA.

In another aspect, the coacervate HA hydrogels of the invention have good moldability properties. In some aspects, the coacervate HA hydrogels have a storage modulus (G') of about 50 Pa to about 5,000 Pa. In other aspects, the coacervate HA hydrogels have a storage modulus of about 500 Pa to about 2,000 Pa, or about 500 Pa to about 1500 Pa, or about 500 Pa to about 1,000 Pa. In other aspects, the coacervate HA hydrogels have a storage modulus of about 500 Pa. In other aspects, the coacervate HA hydrogels have a storage modulus of about 1450.

In another aspect, the coacervate HA hydrogels of the invention are injectable through a needle of at least 18 gauge, more preferably, at least 27 gauge, or an even higher gauge needle. In some aspects, the dermal fillers and coacervate HA hydrogel compositions are injectable through the needle without requiring sizing and/or homogenizing of the composition prior to injection.

Also disclosed are general methods of preparing dermal fillers comprising coacervate HA hydrogels. In some embodiments, the method comprises forming an ionic complex between an anionic HA and a cationic polysaccharide. In some embodiments, the method comprises forming an ionic complex between HA itself and a cationic polysaccharide. In another aspect, the invention provides for methods of preparing coacervate HA hydrogels with different rheological profiles, which may be formed based on the pKa value(s) of the anions and/or cations of each of the anionic HA polysaccharide and the cationic polysaccharide, respectively. In some aspects, the method comprises providing a coacervate HA hydrogel in a physiologically acceptable carrier. In some aspects, the method provides a coacervate HA hydrogel having a storage modulus (G') ranging from about 50 Pa to about 5,000 Pa, for example, from about 500 Pa to about 2,000 Pa, about 500 Pa to about 1500 Pa, or about 500 Pa to about 1,000 Pa, or having a storage modulus of about 500 Pa or about 1450 Pa. In some aspects of the method, the provided dermal filler does not require a further processing step, such as sizing and/or homogenization, prior to injection through a needle, such as a fine needle.

In yet another aspect, the present invention provides non-therapeutic methods of treating a soft tissue of a subject, such as the skin as well the aforementioned dermal filler for use in a method of treating a soft tissue of a subject. In some aspects, the method of treating comprises augmenting the skin or the soft tissue, improving the quality of the skin or soft tissue, or reducing a defect of the skin or soft tissue of the subject. In some aspects, the method comprises the steps of administering (*e.g.,* injecting) a dermal filler of the invention into a subject's soft tissue or skin. In some aspects, the method comprises the step of administering (*e.g.,* injecting) a dermal filler into a dermal region or a hypodermal region of the subject. In some aspects, the method comprises the step of administering (*e.g.,* injecting) a dermal filler into an even a deeper region of a soft tissue (*e.g.,* for volumizing and contouring purposes), of the subject. In some aspects, the treating comprises shaping, filling, volumizing or sculpting the soft tissue or skin of the subject. In other aspects, the treating comprises improving dermal homeostasis, improving skin thickness, healing a wound, or reducing a scar of the subject. In some aspects, the skin defect is a wrinkle, a scar, or a loss of dermal tissue. In some aspects, the treatment is effective for a period of at least about 3 months.

These and other aspects and advantages of the present invention may be more readily understood and appreciated with reference to the following drawings and detailed description.

### BRIEF DESCRIPTION OF THE DRAWINGS

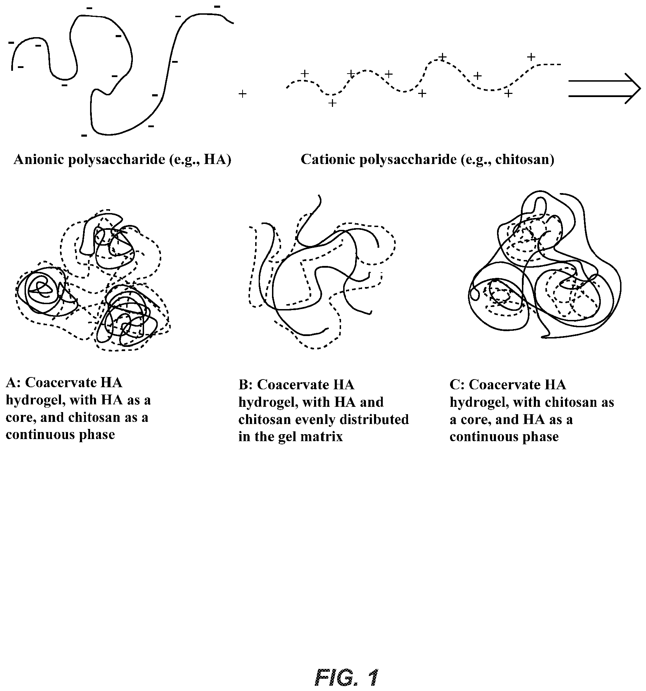

**Figure 1** shows a schematic of a coacervate HA hydrogel formed through non-crosslinked HA and a cationic polysaccharide, such as chitosan. **A:** a non-crosslinked anionic polysaccharide such as HA is condensed or complexed and encapsulated to a core through electrostatic interactions with cationic groups of the cationic polysaccharide, such as chitosan, while the cationic polysaccharide serves as a continuous phase that encapsulates the condensed or complexed anionic HA. Enzymatic degradation of the anionic HA is inhibited, since it is entrapped inside the cationic polysaccharide matrix; for example, reduced accessibility of hyaluronidase protects the HA from hyaluronidase degradation. **B:** the charge ratio between anionic HA and cationic polysaccharide (e.g., chitosan) is close to charge balance; the HA and chitosan form a polyionic complex and are evenly distributed through the gel matrix. **C:** the cationic polysaccharide (e.g., chitosan) is condensed or complexed or encapsulated to a core through electrostatic interactions with anionic groups of the anionic HA, while the anionic HA serves as a continuous phase that encapsulates the condensed or complexed cationic polysaccharide (chitosan). In Figures 1B and 1C, HA forms complexes with the cationic polysaccharide, reducing hyaluronidase accessibility to HA and protecting the HA from hyaluronidase degradation.

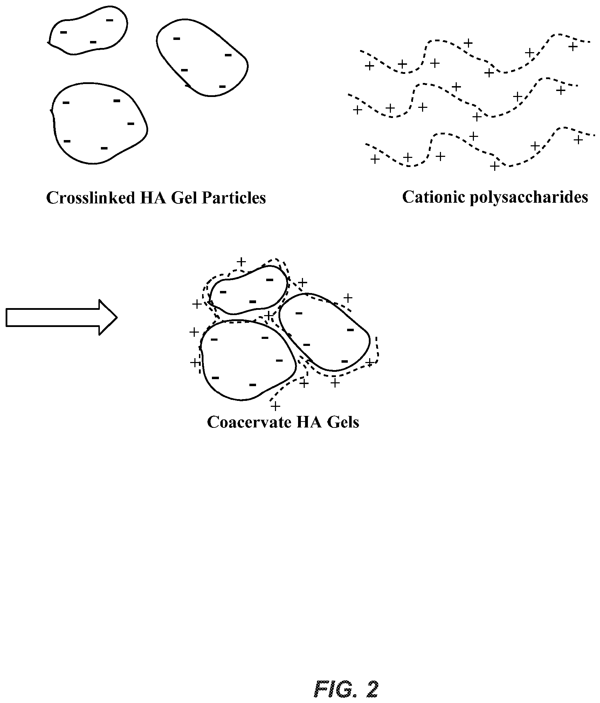

**Figure 2** shows a schematic of a coacervate HA hydrogel formed through crosslinked HA and cationic polysaccharide(s). The crosslinked HA particles are encompassed in a matrix through electrostatic interactions with the cationic groups of the cationic polysaccharide. Because the HA is crosslinked in this case, the HA already has a certain resistance against enzymatic degradation, which may be further increased by being embodied within the coacervate hydrogel. The cationic polysaccharides serve to hold the crosslinked HA gel particles together. The gel has improved cohesivity and moldability as a result of the non-specific (i.e., dynamic or "self-healing") nature of the ionic binding.

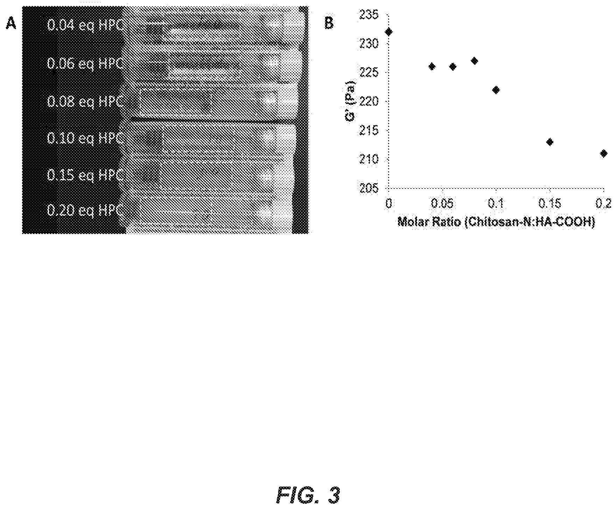

**Figure 3** shows the appearance and mechanical properties of hydrogels prepared with crosslinked HA and varying concentrations of highly pure chitosan (HPC); see Example 4. (A) Hydrogels, prepared in syringes, are outlined with dashed lines. As the HPC content increased from 0.04 eq to 0.20 eq, the hydrogels became more opaque. (B) The hydrogel storage modulus (G') decreased in a stepwise manner as the HPC content increased. The storage modulus is reported for a strain of 1% and frequency of 5 Hz.

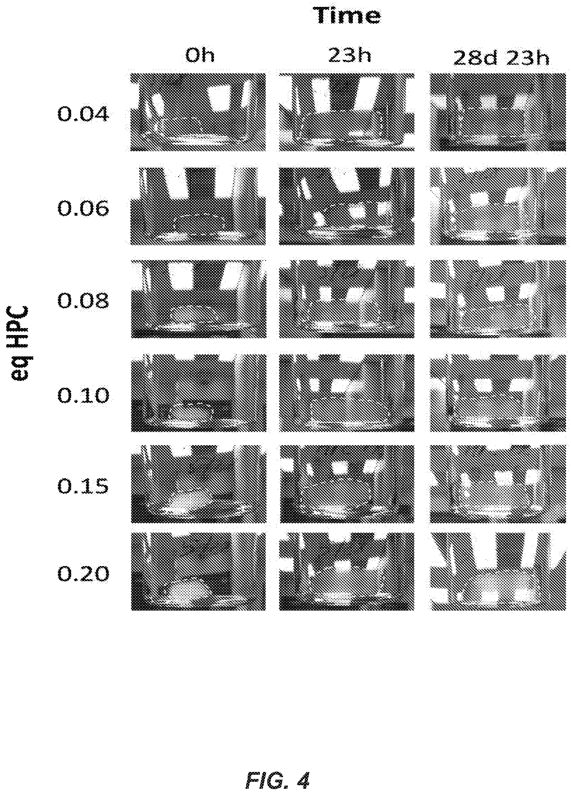

**Figure 4** shows results of the gel swell/dissociation test for the hydrogels of Example 4. The hydrogel (approximately 250 µL) was injected in a cylindrical mold and centrifuged to remove bubbles. The hydrogels were then transferred into PBS (20 mL) and incubated on an orbital shaker at 37°C and 200 RPM. Within the first 24 hours, the hydrogels reached swelling equilibrium and then retained integrity without further dissociation or scattering in PBS. While the gel without added chitosan dissociated in the PBS buffer within two days (not shown), all formulations containing chitosan remained stable for at least 29 days.

### DETAILED DESCRIPTION

The present invention provides for a dermal filler comprising a coacervate HA hydrogel. The hydrogel comprises an ionic complex between an anionic HA and a cationic polysaccharide. In some embodiments, the anionic HA is hyaluronic acid, which may be crosslinked or non-crosslinked.

As used herein, "gel" refers to a nonfluid polymer network that is expanded throughout its whole volume by a fluid.

As used herein, "hydrogel" refers to a nonfluid polymer network that is expanded throughout its whole volume by an aqueous fluid.

As used herein, "coacervate hydrogel" refers to a hydrogel wherein the nonfluid polymer network comprises an ionic complex between an anionic polysaccharide and a cationic polysaccharide, wherein each of the anionic polysaccharide and cationic polysaccharide is independently crosslinked or non-crosslinked. The ionic complex is a noncovalent complex; that is, the anionic polysaccharide and the cationic polysaccharide are not covalently crosslinked to each other.

A coacervate hydrogel of the present invention can be formed by mixing an aqueous composition comprising the anionic polysaccharide with an aqueous composition comprising the cationic polysaccharide, thereby providing a nonfluid polymer network that is expanded throughout its whole volume by an aqueous fluid.

As used herein, "coacervate HA hydrogel" refers to a hydrogel wherein the nonfluid polymer network comprises an ionic complex between an anionic HA polysaccharide and a cationic polysaccharide, wherein each of the anionic HA polysaccharide and cationic polysaccharide is independently crosslinked or non-crosslinked. The ionic complex is a noncovalent complex; that is, the anionic HA polysaccharide and the cationic polysaccharide are not covalently crosslinked to each other.

A coacervate HA hydrogel of the invention can be formed by mixing an aqueous composition comprising the anionic HA polysaccharide with an aqueous composition comprising the cationic polysaccharide, thereby providing the nonfluid polymer network that is expanded throughout its whole volume by an aqueous fluid. The aqueous composition comprising the anionic HA polysaccharide can be prepared from the anionic HA polysaccharide (which may be crosslinked or non-crosslinked) and an aqueous fluid, such as a water, pH-adjusted water (e.g., acidic, neutral, or basic water), or a buffer to give the aqueous composition, which may take a variety of forms, including but not limited to, a solution, a suspension, or a gel. Similarly, the aqueous composition comprising the cationic polysaccharide can be prepared from the cationic polysaccharide (which may be crosslinked or non-crosslinked) and an aqueous fluid, such as a water, pH-adjusted water (e.g., acidic, neutral, or basic water), or a buffer to give the aqueous composition, which may take a variety of forms, including but not limited to, a solution, a suspension, or a gel.

As used herein, "anionic polysaccharide" refers to a polysaccharide having a net negative charge in solution at physiological pH. It is to be understood that reference herein to an anionic polysaccharide does not exclude the presence of one or more neutral or cationic functional groups on the anionic polysaccharide, that is, the anionic polysaccharide need only bear an overall (net) negative charge in solution at physiological pH (or at the pH at which the coacervate complex is formed or used). Specifically, anionic polysaccharide refers to (a) a polysaccharide that is unmodified and comprises a sufficient number of anionic groups such that the overall (net) charge of the polysaccharide is negative at physiological pH; and (b) a polysaccharide that has been modified (i) to comprise a sufficient number of anionic groups such that after the modifying, the overall (net) charge of the polysaccharide is negative at physiological pH; (ii) to change the identity of one or more of the anions of the unmodified anionic polysaccharide, so long as the net charge of the polysaccharide remains negative; (iii) to change (increase or decrease) the number of anions relative to the unmodified polysaccharide, so long as the net charge of the polysaccharide remains negative; and (iv) combinations thereof. Non-limiting examples of anionic polysaccharides include HA, which may be unmodified or may be modified to comprise additional and/or different anionic groups, as disclosed herein. An anionic polysaccharide may be homoanionic or heteroanionic, and may be crosslinked or non-crosslinked.

As used herein, the term "anionic HA" includes both "HA" and a "modified anionic HA."

As used herein, "HA" refers to hyaluronic acid (HA). HA is an anionic polysaccharide, specifically, a glycosaminonglycan. In addition, "HA" refers to hyaluronic acid and any of its hyaluronate salts, including, but not limited to, sodium hyaluronate, potassium hyaluronate, magnesium hyaluronate, calcium hyaluronate, and combinations thereof.

As used herein, the term "modified anionic HA" refers to HA that has been modified to replace one or more carboxylate anions with one or more alternative anions, such as sulfonate and/or phosphonate. A modified anionic HA may be homoanionic or heteroanionic, and may be crosslinked or non-crosslinked.

As used herein, "cationic polysaccharide" refers to an unmodified or modified polysaccharide having a net positive charge in solution at physiological pH (or at the pH at which the coacervate complex is formed or used). Specifically, cationic polysaccharide refers to (a) a polysaccharide that is unmodified and comprises a sufficient number of cationic groups such that the overall (net) charge of the polysaccharide is positive at physiological pH; or (b) a polysaccharide that has been modified (i) to comprise a sufficient number of cationic groups such that after the modifying, the overall charge of the polysaccharide is positive at physiological pH; (ii) to change the identity of one or more of the cations of the unmodified polysaccharide, so long as the net charge of the polysaccharide remains positive; (iii) to change (increase or decrease) the number of cations relative to the unmodified polysaccharide, so long as the net charge of the polysaccharide remains positive; and (iv) combinations thereof. A cationic polysaccharide may be homocationic or heterocationic, and may be crosslinked or non-crosslinked. Non-limiting examples of cationic polysaccharides include chitosan, trimethyl chitosan and cationic HA.

As used herein, "cationic HA" refers to HA that has been modified to comprise a sufficient number of cationic groups such that the overall (net) charge of the resulting polysaccharide is positive at physiological pH. Cationic HA is a cationic polysaccharide. A cationic HA may be homocationic or heterocationic, and may be crosslinked or non-crosslinked.

Non-limiting examples of cationic functional groups include ammonium; guanidinium; heterocyclyl having one or more protonated nitrogen atoms in the ring; and heteroaryl having one or more protonated nitrogen atoms in the ring. As used herein, "ammonium" includes primary ammonium, secondary ammonium, tertiary ammonium, and quaternary ammonium. Specifically, ammonium has the following structure: 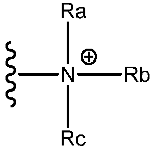 wherein each Ra, Rb, and Rc is independently selected from hydrogen and an unsubstituted or substituted alkyl group, each of which may be the same or different. For example, when each of Ra, Rb and Rc is hydrogen, the cation is a primary ammonium cation; when each of Ra, Rb and Rc is alkyl, the cation is a quaternary ammonium cation.

As used herein, "alkyl" means an aliphatic hydrocarbon group which may be straight or branched and comprising about 1 to about 20 carbon atoms in the chain. Preferred alkyl groups contain about 1 to about 12 carbon atoms in the chain. More preferred alkyl groups contain about 1 to about 6 carbon atoms in the chain. Branched means that one or more lower alkyl groups such as methyl, ethyl or propyl, are attached to a linear alkyl chain. "Alkyl" may be unsubstituted or optionally substituted by one or more substituents which may be the same or different, each substituent being independently selected from the group consisting of halo, alkyl, aryl, heterocyclyl, heteroaryl, cycloalkyl, cyano, hydroxy, alkoxy, alkylthio, amino, oxime (e.g., =N-OH), -NH(alkyl), -NH(cycloalkyl), -N(alkyl)₂, -O-C(O)-alkyl, -O-C(O)-aryl, -O-C(O)-cycloalkyl, -SF₅, carboxy, -C(O)O-alkyl, -C(O)NH(alkyl) and -C(O)N(alkyl)₂. Non-limiting examples of suitable alkyl groups include methyl, ethyl, n-propyl, isopropyl, n-butyl, isobutyl, sec-butyl and t-butyl.

As used herein, "heterocyclyl" means a non-aromatic saturated monocyclic or multicyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination. There are no adjacent oxygen and/or sulfur atoms present in the ring system. Preferred heterocyclyls contain about 5 to about 6 ring atoms. The prefix aza, oxa or thia before the heterocyclyl root name means that at least a nitrogen, oxygen or sulfur atom respectively is present as a ring atom. Any N in a heterocyclyl ring may exist in protonated form. Any -NH in a heterocyclyl ring may exist protected such as, for example, as an -N(Boc), -N(CBz), -N(Tos) group and the like; such protections are also considered part of this invention. The heterocyclyl can be optionally substituted by one or more "ring system substituents" which may be the same or different, and are as defined herein. The nitrogen or sulfur atom of the heterocyclyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, lactam, and the like. "Heterocyclyl" also includes heterocyclyl rings as described above wherein =O replaces two available hydrogens on the same ring carbon atom.

As used herein, "heteroaryl" means an aromatic monocyclic or multicyclic ring system comprising about 5 to about 14 ring atoms, preferably about 5 to about 10 ring atoms, in which one or more of the ring atoms is an element other than carbon, for example nitrogen, oxygen or sulfur, alone or in combination. Preferred heteroaryls contain about 5 to about 6 ring atoms. The prefix aza, oxa or thia before the heterocyclyl root name means that at least a nitrogen, oxygen or sulfur atom respectively is present as a ring atom. Any N in a heterocyclyl ring may exist in protonated form. The "heteroaryl" can be optionally substituted by one or more "ring system substituents" which may be the same or different, and are as defined herein. A nitrogen atom of a heteroaryl can be optionally oxidized to the corresponding N-oxide. Non-limiting examples of suitable heteroaryls include pyridyl, pyrazinyl, pyrimidinyl, pyridone (including N-substituted pyridones), isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like. The term "heteroaryl" also refers to partially saturated heteroaryl moieties such as, for example, tetrahydroisoquinolyl, tetrahydroquinolyl and the like.

As used herein, "ring system substituent" means a substituent attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, aralkyl, alkylaryl, heteroaralkyl, heteroarylalkenyl, heteroarylalkynyl, alkylheteroaryl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylthio, arylthio, heteroarylthio, aralkylthio, heteroaralkylthio, cycloalkyl, heterocyclyl, -SF₅, -O-C(O)-alkyl, -O-C(O)-aryl, -O-C(O)-cycloalkyl, -C(=N-CN)-NH₂, -C(=NH)-NH₂, -C(=NH)-NH(alkyl), oxime (e.g., =N-OH), -NY₁Y₂, -alkyl-NY₁Y₂, -C(O)NY₁Y₂, -SO₂NY₁Y₂ and -SO₂NY₁Y₂, wherein Y₁ and Y₂ can be the same or different and are independently selected from the group consisting of hydrogen, alkyl, aryl, cycloalkyl and aralkyl.

In some embodiments, a cationic functional group of a cationic polysaccharide is provided by incorporating one or more amino acids into the polysaccharide, for example, an arginine sidechain may provide a guanidium ion; a lysine or ornithine sidechain may provide an ammonium ion; a histidine sidechain may provide an imidazolium ion; proline may provide a pyrrolidinium ion.

In other embodiments, the cationic functional group is provided by one or more repeating units of a polysaccharide. In some embodiments, the cationic groups are provided by D-glucosamine, wherein the primary amino groups are protonated to provide primary ammonium ions.

In some embodiments, primary amino groups of a polysaccharide are alkylated to provide the corresponding secondary, tertiary, and/or quaternary ammonium ions.

In some embodiments, the cationic polysaccharide is chitosan. Chitosan, also known as poliglusam, deacetylchitin, and poly-(D)glucosamine, is a linear polysaccharide comprising β-(1-4)-linked D-glucosamine (deacetylated unit) and N-acetyl-D-glucosamine (acetylated unit). Chitosan, including commercially produced chitosan, may be provided by deacetylation of chitin. In some embodiments, the degree of deacetylation (%DD) ranges from about 60 to about 100%. In some embodiments, the amino group of chitosan has a pKa value of about 6.5, which leads to protonation (i.e., the formation of ammonium ions) in acidic to neutral solutions, including physiological pH, wherein the charge density is dependent upon the pH and the %DD. In some embodiments, the molecular weight of chitosan is between about 3800 and about 20,000 Daltons. In other embodiments, the cationic polysaccharide is an alkylamino chitosan. In one such embodiment, the cationic polysaccharide is quaternized chitosan, comprising quaternary ammonium ions. In a particular embodiment, the cationic polysaccharide is trimethyl chitosan.

In some embodiments, a cationic group is introduced into a polysaccharide by derivatizing an anion of the polysaccharide with a group bearing a cation, thereby replacing the anion with a cation. In some embodiments, the polysaccharide is a cationic polysaccharide, and the cation is introduced to incorporate additional cations into the polysaccharide, for example, to modulate the pKa of the cationic polysaccharide, and/or to tune the rheological properties of the coacervate hydrogel comprising the polysaccharide. In other embodiments, the polysaccharide is an anionic polysaccharide, and the cation is introduced to modulate the pKa of the polysaccharide, and/or to tune the rheological properties of the hydrogel comprising the polysaccharide. In some embodiments, the polysaccharide is an anionic polysaccharide, and the cationic groups are introduced to convert the anionic polysaccharide into a cationic polysaccharide. For example, the anionic polysaccharide may be HA, which is converted into cationic HA.

Methods of introducing amine functional groups (i.e., sources of ammonium cations) to a polysaccharide comprising carboxylic acid groups can be achieved by reacting the polysaccharide with a di-amine or polyamine (see Figures 1a and 1b). In some embodiments, the polysaccharide is HA. In an exemplary embodiment, a polysaccharide comprising one or more carboxylic acid groups is coupled with a di-amine or a polyamine in the presence of a coupling agent to form an amide bond. The polysaccharide may be HA. Non-limiting examples of amines useful for introducing cations into a polysaccharide bearing carboxylic acid groups include amino acids such as lysine and ornithine, hexamethylenediamine (HMDA), spermine, spermidine, and derivatives or protected forms of the foregoing. For examples, the amines may comprise one or more carboxylate esters and/or N-Boc groups, such as lysine methyl ester, Nₑₚₛᵢₗₒₙ-Boc-lysine methyl ester, ornithine methyl ester, and N_{delta}-Boc-ornithine methyl ester. Non-limiting examples of coupling agents useful for forming peptide bonds between carboxylic acid groups of a polysaccharide and the amines include carbodiimides, such as dicyclohexylcarbodiimide (DCC), and water soluble carbodiimides, such as 1-ethyl-3-(3-dimethylaminopropyl) carbodiimide (EDC), 1-ethyl-3-(3-trimethylaminopropyl) carbodiimide (ETC), 1-cyclohexyl-3-(2-morpholinoethyl) carbodiimide (CMC), and salts thereof and mixtures thereof.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York. For example, an amino group may be protected with a -(Boc), -(CBz) or -(Tos) group and the like, and subsequently deprotected to provide the corresponding amino group or corresponding ammonium ion.

A non-limiting embodiment of the provided method is depicted in Scheme 1a, which shows the coupling of a polysaccharide comprising carboxylic acid groups with HMDA in the presence of EDC. In this embodiment, one of the HMDA amino groups forms the amide bond, while the other amino group provides an ammonium ion.

Another non-limiting embodiment is depicted in Scheme 1b, which shows the coupling of a polysaccharide comprising carboxylic acid groups with Nₑₚₛᵢₗₒₙ-Boc-lysine methyl ester in the presence of EDC, such that the alpha-amino group of the lysine forms an amide bond with a carboxylic acid group of the polysaccharide; the N-epsilon-BOC group is subsequently deprotected to provide an ammonium ion using methods generally known to one of ordinary skill in the art.

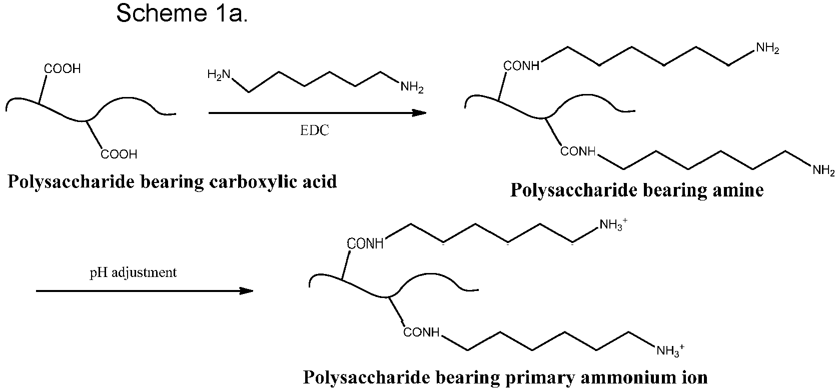

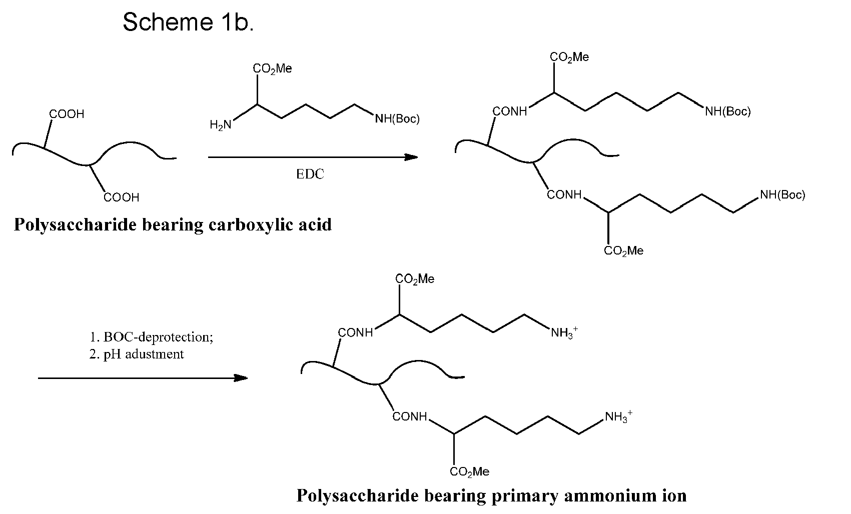

In some embodiments, one or more amino groups of a polysaccharide is derivatized to introduce guanidinium ions. Methods of converting primary ammonium ions to guanidinium ions are disclosed in Hunt, et al. TUNABLE, HIGH MODULUS HYDROGELS DRIVEN BY IONIC COACERVATION, Advanced Materials, 2011, 23, 2327-2331. In one embodiment, a polysaccharide comprising one or more amino/ammonium groups may be reacted with 1H-pyrazole-1-carboximidamide to provide a guanidinium cation, as shown in Schemes 2a and 2b.

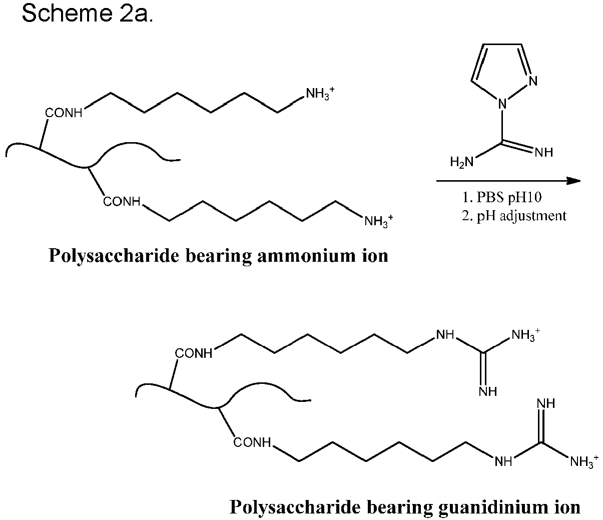

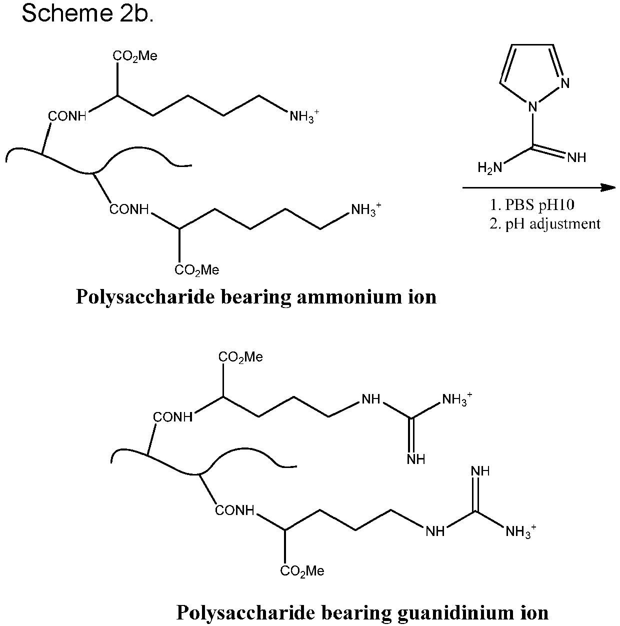

In some embodiments, all of the amino groups of the polysaccharide are converted to guanidinium ions. In other embodiments, only some of the amino groups of the polysaccharide are converted to guanidinium groups, and the resulting polysaccharide comprises both primary ammonium and guinidinium ions.

In another embodiment, all of the amino groups of the polysaccharide are alkylated, e.g., to provide a polysaccharide comprising secondary, tertiary, and/or quaternary ammonium ions. In other embodiments, only some of the amino groups of the polysaccharide are alkylated, and the resulting polysaccharide comprises primary and secondary, tertiary, and/or quaternary ammonium ions.

In another embodiment, some of the amino groups of the polysaccharide are alkylated, and other amino groups are converted to guanidinium ions, thereby providing a polysaccharide comprising primary ammonium ions, secondary ammonium ions, tertiary ammonium ions, quaternary ammonium ions, guanidinium ions, or combinations thereof.

In some embodiments, a crosslinked cationic HA is prepared from crosslinked HA. Non-limiting examples of methods of preparing crosslinked HA are described above. In one embodiment, the crosslinked HA is modified to introduce cationic groups, for example, by incorporating ammonium ions (e.g., see Schemes 1a and 1b), and optionally, by alkylating the ammonium ions, and/or by introducing guanidinium ions (e.g., see Schemes 2a and 2b), thereby providing crosslinked cationic HA bearing primary ammonium, secondary ammonium, tertiary ammonium, quaternary ammonium, and/or guanidinium ions.

In some embodiments, an anionic group is introduced into a polysaccharide. In some embodiments, the polysaccharide is a cationic polysaccharide, and the anion is introduced to incorporate anions into the polysaccharide, for example, to modulate the pKa of the cationic polysaccharide, and/or to tune the rheological properties of the coacervate hydrogel comprising the polysaccharide. In other embodiments, the polysaccharide is HA, and the anion is introduced to modulate the pKa of the HA, and/or to tune the rheological properties of the hydrogel comprising the HA.

Non-limiting examples of anionic functional groups include carboxylate, phosphonate, and sulfonate. In some embodiments, phosphonate and/or sulfonate anions are provided by reacting one or more carboxylate groups of a polysaccharide, such as HA, with aminophosphonic acid to provide a polysaccharide comprising phosphonate ions, and/or with aminosulfamic acid to provide a polysaccharide comprising sulfonate ions, respectively. In some embodiments, the anionic functional group is provided by incorporating an amino acid into the polysaccharide, for example, an aspartatic acid or glutamatic acid sidechain which may provide a carboxylate ion.

It is to be understood that, while the pKa of a particular group may be below 7, the particular group (e.g., imidazole or N-terminal amino) may serve as a cation and therefore be used to form and/or use the coacervate gel under pH conditions at which the group is protonated. Conversely, it is to be understood that, while the pKa of a particular group may be above 7, the particular group may serve as an anion and be used to form and/or use the coacervate gel under pH conditions at which the group is deprotonated.

In some embodiments, there are provided coacervate HA hydrogels with different rheological profiles, which may be formed based on the pKa value(s) of the anions and/or cations of each of the anionic HA and the cationic polysaccharides, respectively. For example, the strength of charge-charge interactions is dependent on the identities of the anions and/or cations, thereby affecting the resulting gel properties. Thus, the rheological properties of the hydrogels can be tuned by selecting particular ionic groups with particular pKa's, and/or by adjusting the relative number of each ionic group. In some embodiments, a homoanionic/homocationic complex, a homoanionic/heterocationic complex, a heteroanionic/homocationic complex, or a heteroanionic/heterocationic complex may be necessary for tuning gel rheological properties.

In one embodiment, the dermal filler of the invention comprises an HA-based hydrogel, the hydrogel comprising an anionic HA and a cationic polysaccharide. In some embodiments, the anionic HA is non-crosslinked. In other embodiments, the anionic HA is crosslinked. In some embodiments, the cationic polysaccharide is non-crosslinked. In other embodiments, the cationic polysaccharide is crosslinked.

In some embodiments, the dermal filler of the invention comprises an HA-based hydrogel, the hydrogel comprising HA and a cationic polysaccharide. In some embodiments, the HA is non-crosslinked. In other embodiments, the HA is crosslinked. In some embodiments, the cationic polysaccharide is non-crosslinked. In other embodiments, the cationic polysaccharide is crosslinked.

In one embodiment, there is provided a coacervate hydrogel comprising a non-crosslinked anionic HA. In this example, the non-crosslinked anionic HA is condensed (i.e., complexed with the cationic polysaccharide) and encapsulated to a core through interactions with cationic groups of the cationic polysaccharide, while the cationic polysaccharide serves as a continuous phase in which the condensed, non-crosslinked anionic HA is dispersed (see **Figure 1A**).

In another embodiment, there is provide a coaervate hydrogel comprising a non-crosslinked HA and non-crosslinked cationic polysaccharide, such as chitosan, in which the charge ratio between anionic HA and cationic chitosan is close to charge balance, HA and chitosan form a polyionic complex and are evenly distributed through the gel matrix (see **Figure 1B**).

In another embodiment, there is provided a coacervate hydrogel comprising non-crosslinked HA and non-crosslinked cationic polysaccharide, such as chitosan, in which the non-crosslinked cationic polysaccharide is condensed (i.e., complexed with an anionic HA) and encapsulated to a core through interactions with anionic groups of the anionic HA, while the anionic HA serves as a continuous phase in which the condensed, non-crosslinked cationic polysaccharide is dispersed (see **Figure 1C**).

In another embodiment, there is provided a coacervate hydrogel comprising a crosslinked anionic HA. In this example, the crosslinked anionic HA polysaccharides are complexed with the cationic polysaccharide, and the cationic polysaccharide serves as a continuous phase in which the crosslinked anioinic HA particles are dispersed (see **Figure 2****).** In some embodiments, the cationic polysaccharides act as cohesive "glues" to hold or encapsulate the crosslinked anionic HA hydrogel particles.

In another embodiment, the hydrogels comprise ionic complexes between non-crosslinked anionic HA and crosslinked cationic HA. The HA serves as a continuous phase in which the crosslinked cationic HA particles are dispersed. In some embodiments, the anionic HA polysaccharides act as cohesive "glues" to hold or encapsulate the crosslinked cationic HA hydrogel particles.

In some embodiments, the coacervate hydrogels comprise a homoanionic HA polysaccharide, wherein each anion has the same identity. For example, the homoanionic polysaccharide may be HA, wherein each anion is a carboxylate anion.

In some embodiments, the hydrogels comprise a modified heteroanionic HA (i.e., HA that has been modified to introduce one or more anions in place of carboxylate), wherein the anions have different identities (e.g., carboxylate, phosphonate, and/or sulfonate), each of which may have pKa values that are the same or different. For example, HA may be reacted with aminophosphonic acid under conditions that provide homoanionic HA having phosphonate anions. In another example, HA may be reacted with aminosulfamic acid under conditions that provide homoanionic HA having sulfonate anions. In another example, HA may be reacted with aminophosphonic acid under conditions that provide heteroanionic HA having both carboxylate and phosphonate anions. In another example, HA may be reacted with aminosulfamic acid to under conditions that provide heteroanionic HA having both carboxylate and sulfonate anions. In another example, HA may be reacted simultaneously or sequentially with aminophosphonic acid and aminosulfamic acid under conditions that provide a heteroanionic HA having carboxylate, phosphonate, and sulfonate groups.

In some embodiments, the coacervate HA hydrogels comprise a homocationic polysaccharide, wherein each cation has the same identity, for example, wherein each cation is a primary ammonium ion. In some embodiments, the homocationic polysaccharide is chitosan.

In other embodiments, the coacervate HA hydrogels comprise a heterocationic polysaccharide, wherein two or more cations have different identities (e.g., primary ammonium, quaternary ammonium, guanidinium, and/or imidazolium), each of which may have pKa values that are the same or different.

In some embodiments, the coacervate hydrogels comprise a homoanionic HA and a homocationic polysaccharide. In some embodiments, the homoanionic HA is HA, and the homocationic polysaccharide is chitosan.

In some embodiments, the coacervate hydrogels comprise a homoanionic HA and a heterocationic polysaccharide. In some embodiments, the homoanionic HA is HA.

In some embodiments, the coacervate hydrogels comprise a heteroanionic HA and a homocationic polysaccharide. In some embodiments, the homocationic polysaccharide is a cationic HA. In some embodiments, the homocationic polysaccharide is chitosan.

In some embodiments, the coacervate hydrogels comprise a heteroanionic HA and a heterocationic polysaccharide.

In some embodiments, the hydrogels comprise an ionic complex between a homoanionic HA and a homocationic polysaccharide. In some embodiments, the homoanionic HA is HA. In some embodiments, the homocationic polysaccharide is a cationic HA. In some embodiments, the homoanionic HA is HA and the homocationic polysaccharide is a cationic HA or chitosan.

In some embodiments, the hydrogels comprise an ionic complex between a homoanionic HA and a heterocationic polysaccharide. In some embodiments, the homoanionic HA is HA.

In some embodiments, the hydrogels comprise an ionic complex between a heteroanionic HA and a homocationic polysaccharide. In some embodiments, the homocationic polysaccharide is a cationic HA or chitosan.

In some embodiments, the hydrogels comprise an ionic complex between a heteroanionic HA and a heterocationic polysaccharide.

Non-limiting embodiments of the invention include hydrogels comprising an ionic complex between an anionic polysaccharide and a cationic polysaccharide, wherein the anionic polysaccharide is selected from the anionic polysaccharide embodiments of Table 1, and the cationic polysaccharide is selected from the cationic polysaccharide embodiments of Table 1, without limitation with respect to the possible combination(s) of anionic and cationic polysaccharides.

**Table 1.**

| **Anionic polysaccharide embodiment** | **Cationic polysaccharide embodiment** |
|---|---|
| a. HA. | a. A cationic polysaccharide |
| b. A modified anionic HA, which is homoanionic or heteranionic. | b. The cationic polysaccharide embodiment a, which is homocationic or heterocation ic. |
| c. The anionic polysaccharide of embodiment b, wherein the anion is selected from carboxylate, sulfonate, phosphonate, and combinations thereof. | c. Cationic HA, which is homocationic or heterocation ic. |
| d. The anionic polysaccharide of any one of embodiments a, b or c, which is crosslinked or non-crosslinked. | d. The cationic polysaccharide of embodiment a, b, or c, wherein the cation is selected from primary ammonium, secondary ammonium, tertiary ammonium, quaternary ammonium, guanidinium, and combinations thereof. |
| | e. The cationic polysaccharide of embodiment a, b, c, or d, which is crosslinked or non-crosslinked. |

In further embodiments, there are provided coacervate HA hydrogels as described in Table 1, the hydrogel further comprising a cosmetic agent, a vitamin, antioxidant, skin-lightening agent, or a combination thereof.

In further embodiments, the coacervate HA hydrogels as described in Table 1 and in the preceding paragraph are provided in a physiologically accceptable carrier, such as, for example, phosphate buffered saline (PBS) or non-crosslinked HA.

In some embodiments, the HA hydrogels of the invention have sufficient or improved moldability, lifting capacity and/or injectability for their intended application as dermal fillers, with improvements relative to conventional chemically-crosslinked HA-based dermal fillers.

In some embodiments, the gels have a storage modulus (G') ranging from about 50 Pa to about 5,000 Pa, for example, from about 500 Pa to about 2000 Pa, about 500 Pa to about 1500 Pa, or about 500 Pa to about 1000 Pa, or have a storage modulus of about 500 Pa or about 1450 Pa. In some aspects, gels with high G' can be obtained by varying the concentrations of the anionic HA and the cationic polysaccharide, the relative ratios of the anionic HA and the cationic polysaccharide, and/or by tuning the anionic-cationic interactions by using different anions and cations to form the complex; for example, the anion-cation pairs may comprise strong anionic-cationic interactions, such as ionic interactions between sulfonate and guanidinium.

In some embodiments of the invention, the coacervate HA hydrogels block accessability to hydrolytic enzymes, leading to better hydrogel integrity, duration, or both, in the soft tissue, such as the skin. In one embodiment, hyaluronidase accessibility is blocked. In another embodiment, enzymes that hydrolyze cationic polysaccharides are blocked. In another embodiment, esterases are blocked.

In some embodiments, the dermal filler of the invention lasts at least about 3 months, at least about 4 months, at least about 5 months, or at least about 6 months after being introduced to the skin or other soft tissue. In some embodiments, the dermal filler of the invention lasts up to about a year after being introduced into the skin or other soft tissue. In other embodiments, the dermal fillers of the invention last up to about 18 months after being introduced into the skin or other soft tissue. In particular embodiments, the dermal filler lasts from between about 3 months and about 18 months after being introduced into the skin or other soft tissue.

In some embodiments, the coacervate HA hydrogels comprise a non-crosslinked anionic HA (such as non-crosslinked HA), having a molecular weight ranging from about 50K to about 3 million Dalton, from about 100K to about 3 million Dalton, from about 500K to about 3 million Dalton, or from about 50K to about 2 million Dalton, from about 100K to about 2 million Dalton, or from about 500 K to about 2 million Dalton.

In some embodiments, the HA component of the hydrogel is a crosslinked anionic HA polysaccharide, such as crosslinked HA. As used herein, the term "crosslinked" refers to the intermolecular bonds joining the individual polymer molecules, or monomer chains, into a more stable structure like a gel. As such, a crosslinked anionic HA polysaccharide has at least one intermolecular bond joining at least one individual polysaccharide molecule to another one.

The crosslinking of glycosaminoglycan polysaccharides, such as HA, typically result in the formation of a hydrogel. Such hydrogels have high viscosity and require considerable force to extrude through a fine needle. Glycosaminoglycan polysaccharides in general, including HA, may be crosslinked using dialdehydes and disufides crosslinking agents including, without limitation, multifunctional PEG-based crosslinking agents, divinyl sulfones, diglycidyl ethers, and bis-epoxides, biscarbodiimide. Non-limiting examples of HA crosslinking agents include multifunctional PEG-based crosslinking agents like pentaerythritol tetraglycidyl ether (PETGE), divinyl sulfone (DVS), 1,4-butanediol diglycidyl ether (BDDE), 1,2-bis(2,3-epoxypropoxy)ethylene (EGDGE), 1,2,7,8-diepoxyoctane (DEO), (phenylenebis-(ethyl)-carbodiimide and 1,6 hexamethylenebis (ethylcarbodiimide), adipic dihydrazide (ADH), bis(sulfosuccinimidyl)suberate (BS), hexamethylenediamine (HMDA), 1-(2,3-epoxypropyl)-2,3-epoxycyclohexane, or combinations thereof. Other useful cross-linking agents are disclosed in Stroumpoulis and Tezel, Tunably Crosslinked Polysaccharide Compositions, U.S. Patent Publication US 2011/0077737. Non-limiting examples of methods of crosslinking glycosaminoglycan polysaccharides are described in, e.g., Piron and Tholin, Polysaccharide Crosslinking, Hydrogel Preparation, Resulting Polysaccharides(s) and Hydrogel(s), uses Thereof, U.S. Patent Publication 2003/0148995; Lebreton, Cross-Linking of Low and High Molecular Weight Polysaccharides, Preparation of Injectable Monophase Hydrogels, Polysaccharides and Hydrogels Obtained, U.S. Patent Publication 2010/0226988; Lebreton, Viscoelastic Solutions Containing Sodium Hyaluronate and Hydroxypropyl Methyl Cellulose, Preparation and Uses, U.S. Patent Publication 2008/0089918; Lebreton, Hyaluronic Acid-Based Gels Including Lidocaine, U.S. Patent Publication 2010/0028438; and Polysaccharides and Hydrogels thus Obtained, U.S. Patent Publication 2006/0194758; and Di Napoli, Composition and Method for Intradermal Soft Tissue Augmentation, International Patent Publication WO 2004/073759; Njikang et al., Dermal Filler Compositions, U.S. Patent Publication 2013/0096081.

In some embodiments, crosslinked modified anionic HA polysaccharides are prepared from crosslinked HA. Non-limiting examples of methods of preparing crosslinked HA are described above. In one embodiment, a crosslinked HA is modified to introduce anionic groups in addition to or in place of carboxylate. For example, crosslinked HA may be reacted with aminophosphonic acid to provide crosslinked homoanionic HA having phosphonate anions. In another example, crosslinked HA may be reacted with aminosulfamic acid to provide crosslinked homoanionic HA having sulfonate anions. In another example, crosslinked HA may be reacted with aminophosphonic acid to provide crosslinked heteroanionic HA having both carboxylate and phosphonate anions. In another example, crosslinked HA may be reacted with aminosulfamic acid to provide crosslinked heteroanionic HA having both carboxylate and sulfonate anions. In another example, crosslinked HA may be reacted simultaneously or sequentially with aminophosphonic acid and aminosulfamic acid to provide a crosslinked heteroanionic HA having carboxylate, phosphonate, and sulfonate groups.

In accordance with the present specification, "%" in a formulation is defined as weight by weight (i.e., w/w) percentage. As an example: 1% (w/w) means a concentration of 10 mg/g.

In an embodiment, a hydrogel composition comprises an anionic HA polysaccharide (such as HA) which is present in an amount sufficient to treat a soft tissue or skin condition as disclosed herein. In other aspects of this embodiment, a composition comprises an anionic HA (such as HA) which represents, e.g., about 1% by weight, about 2% by weight, about 3% by weight, about 4% by weight, about 5% by weight, about 6% by weight, about 7% by weight, about 8% by weight, or about 9%, or about 10% by weight, of the total composition. In yet other aspects of this embodiment, a composition comprises an anionic HA (such as HA) represents, e.g., at most 1% by weight, at most 2% by weight, at most 3% by weight, at most 4% by weight, at most 5% by weight, at most 6% by weight, at most 7% by weight, at most 8% by weight, at most 9% by weight, or at most 10% by weight, of the total composition. In still other aspects of this embodiment, a composition comprises an anionic HA (such as HA) which represents, e.g., about 0.5% to about 20% by weight, about 1% to about 17% by weight, about 3% to about 15% by weight, or about 5% to about 10% by weight, for example, about 11% by weight, about 15% by weight or about 17% by weight, of the total composition.

In aspects of this embodiment, a hydrogel composition comprises an anionic HA (such as HA) which is present at a concentration of, e.g., about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, or about 20 mg/g. In other aspects of this embodiment, a composition comprises an anionic HA (such as HA) which is present at a concentration of, e.g., at least 1 mg/g, at least 2 mg/g, at least 3 mg/g, at least 4 mg/g, at least 5 mg/g, at least 10 mg/g, at least 15 mg/g, at least 20 mg/g, or at least 25 mg/g, or about 40 mg/g. In yet other aspects of this embodiment, a composition comprises an anionic HA (such as HA) which is present at a concentration of, e.g., at most 1 mg/g, at most 2 mg/g, at most 3 mg/g, at most 4 mg/g, at most 5 mg/g, at most 10 mg/g, at most 15 mg/g, at most 20 mg/g, at most 25 mg/g, or at most 40 mg/g. In still other aspects of this embodiment, a composition comprises an anionic HA (such as HA) which is present at a concentration of, e.g., about 7.5 mg/g to about 19.5 mg/g, about 8.5 mg/g to about 18.5 mg/g, about 9.5 mg/g to about 17.5 mg/g, about 10.5 mg/g to about 16.5 mg/g, about 11.5 mg/g to about 15.5 mg/g, or about 12.5 mg/g to about 14.5 mg/g, or up to about 40 mg/g.

Aspects of the present specification provide, in part, a hydrogel composition comprising a crosslinked anionic HA polysaccharide having a degree of crosslinking. As used herein, the term "degree of crosslinking" refers to the percentage of anionic HA polysaccharide monomeric units, such as, e.g., the disaccharide monomer units of HA, that are bound to a cross-linking agent. The degree of crosslinking is expressed as the percent weight ratio of the crosslinking agent to anionic HA. In some embodiments, coacervate hydrogels of the invention comprise a crosslinked anionic HA having a degree of crosslinking from about 1 wt% to about 15 wt%, about 1 wt% to about 10 wt%, about 2 wt% to about 10 wt%, about 3 wt% to about 10 wt% or about 5 wt% to about 10 wt%. In other embodiments, coacervate hydrogels of the invention comprise a crosslinked anionic HA having a degree of crosslinking of less than about 10%.

Aspects of the present specification provide, in part, a hydrogel composition comprising a non-crosslinked anionic HA polysaccharide. As used herein, the term "non-crosslinked" refers to a lack of intermolecular bonds joining the individual anionic polysaccharide molecules, or monomer chains. As such, a non-crosslinked anionic HA polysaccharide (including non-crosslinked HA) is not linked to any other anionic HA polysaccharide by a covalent intermolecular bond. In aspects of this embodiment, a dermal filler composition comprises a non-crosslinked anionic HA polysaccharide, such as non-crosslinked HA. In other aspects, the dermal filler comprises a coacervate HA hydrogel, wherein the coacervate complex comprises the non-crosslinked anionic HA polysaccharide, such as non-crosslinked HA.

Non-crosslinked HA polysaccharides are water soluble and generally remain fluid in nature. As such, non-crosslinked HA polysaccharides can be mixed with a coacervate HA polysaccharide-based hydrogel as a lubricant to facilitate the extrusion process of the composition through a fine needle.

Aspects of the present invention provide, in part, a hydrogel composition comprising anionic HA polysaccharides of low molecular weight, anionic HA polysaccharides of high molecular weight, or anionic HA polysaccharides of both low and high molecular weight. As used herein, the term "high molecular weight" when referring to "anionic HA" refers to anionic HA polysaccharides (including HA) having a mean molecular weight of 1,000,000 Da or greater. Non-limiting examples of a high molecular weight anionic HA polysaccharides (including HA) are those of about 1,500,000 Da, about 2,000,000 Da, about 2,500,000 Da, about 3,000,000 Da, about 3,500,000 Da, about 4,000,000 Da, about 4,500,000 Da, or about 5,000,000 Da. As used herein, the term "low molecular weight" when referring to "anionic HA" refers to anionic HA polysaccharides (including HA) having a mean molecular weight of less than 1,000,000 Da. Non-limiting examples of a low molecular weight anionic HA polysaccharides (such as HA) are those of about 100,000 Da, about 200,000 Da, about 300,000 Da, about 400,000 Da, about 500,000 Da, about 600,000 Da, about 700,000 Da, of about 800,000 Da, or about 900,000 Da.

In an embodiment, a dermal filler composition comprises non-crosslinked low molecular weight anionic HA polysaccharides, or comprises crosslinked anionic HA polysaccharides prepared from non-crosslinked low molecular weight anionic HA, wherein the non-crosslinked low molecular weight anionic HA has mean molecular weight of, e.g., about 100,000 Da, about 200,000 Da, about 300,000 Da, about 400,000 Da, about 500,000 Da, about 600,000 Da, about 700,000 Da, about 800,000 Da, or about 900,000 Da. In yet other aspects of this embodiment, the non-crosslinked low molecular weight anionic HA has a mean molecular weight of, e.g., at most 100,000 Da, at most 200,000 Da, at most 300,000 Da, at most 400,000 Da, at most 500,000 Da, at most 600,000 Da, at most 700,000 Da, at most 800,000 Da, at most 900,000 Da, or at most 950,000. In still other aspects of this embodiment, the non-crosslinked low molecular weight HA has a mean molecular weight of, e.g., about 100,000 Da to about 500,000 Da, about 200,000 Da to about 500,000 Da, about 300,000 Da to about 500,000 Da, about 400,000 Da to about 500,000 Da, about 500,000 Da to about 950,000 Da, about 600,000 Da to about 950,000 Da, about 700,000 Da to about 950,000 Da, about 800,000 Da to about 950,000 Da, about 300,000 Da to about 600,000 Da, about 300,000 Da to about 700,000 Da, about 300,000 Da to about 800,000 Da, or about 400,000 Da to about 700,000 Da.

In another embodiment, a composition comprises non-crosslinked high molecular weight anionic HA polysaccharides (such as non-crosslinked HA), or comprises crosslinked anionic HA polysaccharides prepared from non-crosslinked high molecular weight HA, wherein the non-crosslinked high molecular weight anionic HA has a mean molecular weight of, e.g., about 1,000,000 Da, about 1,500,000 Da, about 2,000,000 Da, about 2,500,000 Da, about 3,000,000 Da, about 3,500,000 Da, about 4,000,000 Da, about 4,500,000 Da, or about 5,000,000 Da. In other aspects of this embodiment, the non-crosslinked high molecular weight anionic HA has a mean molecular weight of, e.g., at least 1,000,000 Da, at least 1,500,000 Da, at least 2,000,000 Da, at least 2,500,000 Da, at least 3,000,000 Da, at least 3,500,000 Da, at least 4,000,000 Da, at least 4,500,000 Da, or at least 5,000,000 Da. In yet other aspects of this embodiment, the non-crosslinked high molecular weight anionic HA has a mean molecular weight of, e.g., about 1,000,000 Da to about 5,000,000 Da, about 1,500,000 Da to about 5,000,000 Da, about 2,000,000 Da to about 5,000,000 Da, about 2,500,000 Da to about 5,000,000 Da, about 2,000,000 Da to about 3,000,000 Da, about 2,500,000 Da to about 3,500,000 Da, or about 2,000,000 Da to about 4,000,000 Da. In still other aspects, the non-crosslinked high molecular weight anionic HA has a mean molecular weight of e.g., greater than 2,000,000 Da and less than about 3,000,000 Da, greater than 2,000,000 Da and less than about 3,500,000 Da, greater than 2,000,000 Da and less than about 4,000,000 Da, greater than 2,000,000 Da and less than about 4,500,000 Da, greater than 2,000,000 Da and less than about 5,000,000 Da.

In another embodiment, a dermal filler comprises a combination of both high molecular weight anionic HA polysaccharides and low molecular weight anionic HA polysaccharides, in various ratios; for example, the ratio of high molecular weight anionic HA to low molecular weight anionic HA may be about 20:1, about 15:1, about 10:1, about 5:1, about 1:1, about 1:5 about 1:10, about 1:15, or about 1:20. In some embodiments, the high molecular weight anionic HA polysaccharide is crosslinked with the low molecular weight anionic HA polysaccharide in the foregoing ratios.

In some embodiments, the cationic polysaccharide of the coacervate hydrogel is present in an amount sufficient to treat a skin condition as disclosed herein. In other aspects of this embodiment, a composition comprises a cationic polysaccharide representing, e.g., about 1% by weight, about 2% by weight, about 3% by weight, about 4% by weight, about 5% by weight, about 6% by weight, about 7% by weight, about 8% by weight, or about 9%, or about 10% by weight, of the total composition. In yet other aspects of this embodiment, a composition comprises a cationic polysaccharide representing, e.g., at most 1% by weight, at most 2% by weight, at most 3% by weight, at most 4% by weight, at most 5% by weight, at most 6% by weight, at most 7% by weight, at most 8% by weight, at most 9% by weight, or at most 10% by weight, of the total composition. In still other aspects of this embodiment, a composition comprises a cationic polysaccharide representing, e.g., about 0.5% to about 20% by weight, about 1% to about 17% by weight, about 3% to about 15% by weight, or about 5% to about 10% by weight, for example, about 11% by weight, about 15% by weight or about 17% by weight, of the total composition.

In aspects of this embodiment, a hydrogel composition comprises a cationic polysaccharide which is present at a concentration of, e.g., about 2 mg/g, about 3 mg/g, about 4 mg/g, about 5 mg/g, about 6 mg/g, about 7 mg/g, about 8 mg/g, about 9 mg/g, about 10 mg/g, about 11 mg/g, about 12 mg/g, about 13 mg/g, about 13.5 mg/g, about 14 mg/g, about 15 mg/g, about 16 mg/g, about 17 mg/g, about 18 mg/g, about 19 mg/g, or about 20 mg/g. In other aspects of this embodiment, a composition comprises a cationic polysaccharide which is present at a concentration of, e.g., at least 1 mg/g, at least 2 mg/g, at least 3 mg/g, at least 4 mg/g, at least 5 mg/g, at least 10 mg/g, at least 15 mg/g, at least 20 mg/g, or at least 25 mg/g, or about 40 mg/g. In yet other aspects of this embodiment, a composition comprises a cationic polysaccharide which is present at a concentration of, e.g., at most 1 mg/g, at most 2 mg/g, at most 3 mg/g, at most 4 mg/g, at most 5 mg/g, at most 10 mg/g, at most 15 mg/g, at most 20 mg/g, at most 25 mg/g, or at most 40 mg/g. In still other aspects of this embodiment, a composition comprises a cationic polysaccharide which is present at a concentration of, e.g., about 7.5 mg/g to about 19.5 mg/g, about 8.5 mg/g to about 18.5 mg/g, about 9.5 mg/g to about 17.5 mg/g, about 10.5 mg/g to about 16.5 mg/g, about 11.5 mg/g to about 15.5 mg/g, or about 12.5 mg/g to about 14.5 mg/g, or up to about 40 mg/g.

Aspects of the present specification provide, in part, a hydrogel composition comprising a crosslinked cationic polysaccharide having a degree of crosslinking. As used herein, the term "degree of crosslinking" refers to the percentage of cationic polysaccharide monomeric units, such as, e.g., the disaccharide monomer units of cationic polysaccharide that are bound to a cross-linking agent. The degree of crosslinking is expressed as the percent weight ratio of the crosslinking agent to cationic HA. In some embodiments, coacervate hydrogels of the invention comprise a crosslinked cationic polysaccharide having a degree of crosslinking from about 1 wt% to about 15 wt%, about 1 wt% to about 10 wt%, about 2 wt% to about 10 wt%, about 3 wt% to about 10 wt% or about 5 wt% to about 10wt%. In other embodiments, coacervate hydrogels of the invention comprise a crosslinked cationic polysaccharide having a degree of crosslinking of less than about 10%.

Aspects of the present specification provide, in part, a hydrogel composition comprising an non-crosslinked cationic polysaccharide. As used herein, the term "non-crosslinked" refers to a lack of intermolecular bonds joining the individual cationic polysaccharide molecules, or monomer chains. As such, a non-crosslinked cationic polysaccharide is not linked to any other cationic polysaccharide by a covalent intermolecular bond. In aspects of this embodiment, a dermal filler composition comprises a non-crosslinked cationic polysaccharide. In other aspects, the dermal filler comprises a coacervate HA hydrogel, wherein the coacervate complex comprises the non-crosslinked cationic polysaccharide.

Aspects of the present invention provide, in part, a hydrogel composition comprising cationic polysaccharides of low molecular weight, of high molecular weight, or of both low and high molecular weight. As used herein, the term "high molecular weight" when referring to "cationic polysaccharides" refers to those having a mean molecular weight of 1,000,000 Da or greater. Non-limiting examples of a high molecular weight cationic polysaccharides include those of about 1,500,000 Da, about 2,000,000 Da, about 2,500,000 Da, about 3,000,000 Da, about 3,500,000 Da, about 4,000,000 Da, about 4,500,000 Da, and about 5,000,000 Da. As used herein, the term "low molecular weight" when referring to "cationic polysaccharides" refers to those having a mean molecular weight of less than 1,000,000 Da. Non-limiting examples of a low molecular weight cationic polysaccharides include those of about 100,000 Da, about 200,000 Da, about 300,000 Da, about 400,000 Da, about 500,000 Da, about 600,000 Da, about 700,000 Da, of about 800,000 Da, and about 900,000 Da.

In an embodiment, a dermal filler composition comprises non-crosslinked low molecular weight cationic polysaccharides, or crosslinked cationic polysaccharides prepared from non-crosslinked low molecular weight cationic polysaccharides, wherein the non-crosslinked low molecular weight cationic polysaccharide has a mean molecular weight of, e.g., about 100,000 Da, about 200,000 Da, about 300,000 Da, about 400,000 Da, about 500,000 Da, about 600,000 Da, about 700,000 Da, about 800,000 Da, or about 900,000 Da. In yet other aspects of this embodiment, the non-crosslinked low molecular weight cationic polysaccharide has a mean molecular weight of, e.g., at most 100,000 Da, at most 200,000 Da, at most 300,000 Da, at most 400,000 Da, at most 500,000 Da, at most 600,000 Da, at most 700,000 Da, at most 800,000 Da, at most 900,000 Da, or at most 950,000. In still other aspects of this embodiment, the non-crosslinked low molecular weight cationic polysaccharide has a mean molecular weight of, e.g., about 100,000 Da to about 500,000 Da, about 200,000 Da to about 500,000 Da, about 300,000 Da to about 500,000 Da, about 400,000 Da to about 500,000 Da, about 500,000 Da to about 950,000 Da, about 600,000 Da to about 950,000 Da, about 700,000 Da to about 950,000 Da, about 800,000 Da to about 950,000 Da, about 300,000 Da to about 600,000 Da, about 300,000 Da to about 700,000 Da, about 300,000 Da to about 800,000 Da, or about 400,000 Da to about 700,000 Da.

In another embodiment, a composition comprises non-crosslinked high molecular weight cationic polysaccharides, or crosslinked cationic polysaccharides prepared from non-crosslinked high molecular weight cationic polysaccharides, wherein the non-crosslinked high molecular weight cationic polysaccharide has a mean molecular weight of, e.g., about 1,000,000 Da, about 1,500,000 Da, about 2,000,000 Da, about 2,500,000 Da, about 3,000,000 Da, about 3,500,000 Da, about 4,000,000 Da, about 4,500,000 Da, or about 5,000,000 Da. In other aspects of this embodiment, the non-crosslinked high molecular weight cationic polysaccharide has a mean molecular weight of, e.g., at least 1,000,000 Da, at least 1,500,000 Da, at least 2,000,000 Da, at least 2,500,000 Da, at least 3,000,000 Da, at least 3,500,000 Da, at least 4,000,000 Da, at least 4,500,000 Da, or at least 5,000,000 Da. In yet other aspects of this embodiment, the non-crosslinked high molecular weight cationic polysaccharide has a mean molecular weight of, e.g., about 1,000,000 Da to about 5,000,000 Da, about 1,500,000 Da to about 5,000,000 Da, about 2,000,000 Da to about 5,000,000 Da, about 2,500,000 Da to about 5,000,000 Da, about 2,000,000 Da to about 3,000,000 Da, about 2,500,000 Da to about 3,500,000 Da, or about 2,000,000 Da to about 4,000,000 Da. In still other aspects, the non-crosslinked high molecular weight cationic polysaccharide has a mean molecular weight of, e.g., greater than 2,000,000 Da and less than about 3,000,000 Da, greater than 2,000,000 Da and less than about 3,500,000 Da, greater than 2,000,000 Da and less than about 4,000,000 Da, greater than 2,000,000 Da and less than about 4,500,000 Da, greater than 2,000,000 Da and less than about 5,000,000 Da.

In another embodiment, a dermal filler comprising a combination of both high molecular weight cationic polysaccharides and low molecular weight cationic polysaccharides, in various ratios. In aspects of this embodiment, a dermal filler comprises a combination of both high molecular weight cationic polysaccharides and low molecular weights in a ratio of about 20:1, about 15:1, about 10:1, about 5:1, about 1:1, about 1:5 about 1:10, about 1:15, or about 1:20. In some embodiments, the high molecular weight cationic polysaccharide is crosslinked with the low molecular weight cationic polysaccharide in the foregoing ratios.

A dermal filler composition or coacervate HA hydrogel disclosed herein may further comprise another agent or combination of agents that provide a beneficial effect when the composition is administered to an individual. Such beneficial agents include, without limitation, cosmetic agents or other ingredients. Non-limiting examples of such beneficial agents include an antioxidant, an anti-itching agent, an anti-cellulite agent, an anti-scarring agent, an anti-inflammatory agent, an anesthetic agent, an anti-irritant agent, a desquamating agent, a tensioning agent, an anti-acne agent, a skin-lightening agent, a pigmentation agent, an anti-pigmentation agent, a moisturizing agent, or a vitamin. In some embodiments, the agent is added and trapped within the coacervate without modification of the agent, thus providing means to slowly release and deliver the agent. A significant advantage of this approach is that it incorporates the beneficial agents without crosslinking them to the coacervate HA gel complex so that the components can be captured in their native state with a tuned rate of release controlled by the density, rate of diffusion, rate of degradation, and/or rheologic properties of the HA coacervate gel. In some embodiments, the agent forms ionic or electrostatic interactions with one or more polysaccharides of the coacervate HA hydrogel. In some embodiments, the release of the agent is controlled by the ionic or electrostatic interactions between the agent and the one or more of the coacervate HA polysaccharides. In a further embodiment, the coacervate HA complex is tuned for faster or slower release of the agent; for example, the number, identity, and/or pKa values of the polysaccharides of the coacervate HA complex may be tuned for faster or slower release of the agent. The release profile can be days, or weeks, or months, or even longer to achieve the desirable benefits.

Aspects of the present specification provide, in part, a dermal filler composition that may optionally comprise an anesthetic agent or salt thereof. An anesthetic agent is preferably a local anesthetic agent, i.e., an anesthetic agent that causes a reversible local anesthesia and a loss of nociception, such as, e.g., aminoamide local anesthetics and aminoester local anesthetics. The amount of an anesthetic agent included in a composition disclosed herein is an amount effective to mitigate pain experienced by an individual upon administration of the dermal filler composition. As such, the amount of an anesthetic agent included in a dermal filler composition disclosed in the present specification is between about 0.1% to about 5% by weight of the total composition. Non-limiting examples of anesthetic agents include lidocaine, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, combinations thereof, and salts thereof. Non-limiting examples of aminoester local anesthetics include procaine, chloroprocaine, cocaine, cyclomethycaine, cimethocaine (larocaine), propoxycaine, procaine (novocaine), proparacaine, tetracaine (amethocaine). Non-limiting examples of aminoamide local anesthetics include articaine, bupivacaine, cinchocaine (dibucaine), etidocaine, levobupivacaine, lidocaine (lignocaine), mepivacaine, piperocaine, prilocaine, ropivacaine, and trimecaine. A composition disclosed herein may comprise a single anesthetic agent or a plurality of anesthetic agents. A non-limiting example of a combination local anesthetic is lidocaine/prilocaine (EMLA).

In other aspects of this embodiment, a dermal filler composition disclosed herein comprises an anesthetic agent in an amount of, e.g., about 0.1%, about 0.2%, about 0.3%, about 0.4%, about 0.5%, about 0.6%, about 0.7%, about 0.8% about 0.9%, about 1.0%, about 2.0%, about 3.0%, about 4.0%, about 5.0%, about 6.0%, about 7.0%, about 8.0%, about 9.0%, or about 10% by weight of the total composition. In yet other aspects, a composition disclosed herein comprises an anesthetic agent in an amount of, e.g., at least 0.1%, at least 0.2%, at least 0.3%, at least 0.4%, at least 0.5%, at least 0.6%, at least 0.7%, at least 0.8% at least 0.9%, at least 1.0%, at least 2.0%, at least 3.0%, at least 4.0%, at least 5.0%, at least 6.0%, at least 7.0%, at least 8.0%, at least 9.0%, or at least 10% by weight of the total composition. In still other aspects, a dermal filler composition disclosed herein comprises an anesthetic agent in an amount of, e.g., at most 0.1%, at most 0.2%, at most 0.3%, at most 0.4%, at most 0.5%, at most 0.6%, at most 0.7%, at most 0.8% at most 0.9%, at most 1.0%, at most 2.0%, at most 3.0%, at most 4.0%, at most 5.0%, at most 6.0%, at most 7.0%, at most 8.0%, at most 9.0%, or at most 10% by weight of the total composition. In further aspects, a dermal filler composition disclosed herein comprises an anesthetic agent in an amount of, e.g., about 0.1% to about 0.5%, about 0.1% to about 1.0%, about 0.1% to about 2.0%, about 0.1% to about 3.0%, about 0.1% to about 4.0%, about 0.1% to about 5.0%, about 0.2% to about 0.9%, about 0.2% to about 1.0%, about 0.2% to about 2.0%, about 0.5% to about 1.0%, or about 0.5% to about 2.0% by weight of the total composition.

In another embodiment, a dermal filler disclosed herein does not comprise an anesthetic agent.

Aspects of the present specification provide, in part, a coacervate HA hydrogel composition that exhibits a complex modulus, an elastic modulus, a viscous modulus and/or a tan δ. The compositions as disclosed herein are viscoelastic in that the composition has an elastic component (solid-like such as, e.g., crosslinked anionic or cationic HA polysaccharides) and a viscous component (liquid-like such as, e.g., uncrosslinked HA polysaccharides or a carrier phase) when a force is applied (stress, deformation). The rheological attribute that describes this property is the complex modulus (G*), which defines a composition's total resistance to deformation. The complex modulus is a complex number with a real and imaginary part: G*=G'+iG". The absolute value of G* is Abs(G*) = Sqrt(G'2+G"2). The complex modulus can be defined as the sum of the elastic modulus (G') and the viscous modulus (G"). Falcone, et al., Temporary Polysaccharide Dermal Fillers: A Model for Persistence Based on Physical Properties, Dermatol Surg. 35(8): 1238-1243 (2009), which is hereby incorporated by reference in its entirety.

Elastic modulus, or modulus of elasticity, refers to the ability of a hydrogel material to resist deformation, or, conversely, an object's tendency to be non-permanently deformed when a force is applied to it. Elastic modulus characterizes the firmness of a composition and is also known as the storage modulus because it describes the storage of energy from the motion of the composition. The elastic modulus describes the interaction between elasticity and strength (G' = stress/strain) and, as such, provides a quantitative measurement of a composition's hardness or softness. The elastic modulus of an object is defined as the slope of its stress-strain curve in the elastic deformation region: λ = stress/strain, where λ is the elastic modulus in Pascal's; stress is the force causing the deformation divided by the area to which the force is applied; and strain is the ratio of the change caused by the stress to the original state of the object. Although depending on the speed at which the force is applied, a stiffer composition will have a higher elastic modulus and it will take a greater force to deform the material a given distance, such as, e.g., an injection. Specifying how stresses are to be measured, including directions, allows for many types of elastic moduli to be defined. The three primary elastic moduli are tensile modulus, shear modulus, and bulk modulus.

Viscous modulus is also known as the loss modulus because it describes the energy that is lost as viscous dissipation. Tan δ is the ratio of the viscous modulus and the elastic modulus, tan δ = G"/G'. Falcone, supra, 2009. For tan δ values disclosed in the present specification, a tan δ is obtained from the dynamic modulus at a frequency of 1 Hz. A lower tan δ corresponds to a stiffer, harder, or more elastic composition.

In another embodiment, a coacervate HA hydrogel composition disclosed herein exhibits an elastic modulus of, e.g., about 25 Pa, about 50 Pa, about 75 Pa, about 100 Pa, about 125 Pa, about 150 Pa, about 175 Pa, about 200 Pa, about 250 Pa, about 300 Pa, about 350 Pa, about 400 Pa, about 450 Pa, about 500 Pa, about 550 Pa, about 600 Pa, about 650 Pa, about 700 Pa, about 750 Pa, about 800 Pa, about 850 Pa, about 900 Pa, about 950 Pa, about 1,000 Pa, about 1,200 Pa, about 1,300 Pa, about 1,400 Pa, about 1450 Pa, about 1,500 Pa, about 1,600 Pa, about 1700 Pa, about 1800 Pa, about 1900 Pa, about 2,000 Pa, about 2,100 Pa, about 2,200 Pa, about 2,300 Pa, about 2,400 Pa, about 2,500 Pa, about 3000 Pa, about 4000 Pa, about 5000 Pa, about 6000 Pa, about 7000 Pa, about 8000 Pa, about 9000 Pa, or about 10,000 Pa. In other aspects of this embodiment, a hydrogel composition exhibits an elastic modulus of, e.g., at least 25 Pa, at least 50 Pa, at least 75 Pa, at least 100 Pa, at least 125 Pa, at least 150 Pa, at least 175 Pa, at least 200 Pa, at least 250 Pa, at least 300 Pa, at least 350 Pa, at least 400 Pa, at least 450 Pa, at least 500 Pa, at least 550 Pa, at least 600 Pa, at least 650 Pa, at least 700 Pa, at least 750 Pa, at least 800 Pa, at least 850 Pa, at least 900 Pa, at least 950 Pa, at least 1,000 Pa, at least 1,200 Pa, at least 1,300 Pa, at least 1,400 Pa, at least 1,500 Pa, at least 1,600 Pa, at least 1700 Pa, at least 1800 Pa, at least 1900 Pa, at least 2,000 Pa, at least 2,100 Pa, at least 2,200 Pa, at least 2,300 Pa, at least 2,400 Pa, or at least 2,500 Pa. In yet other aspects of this embodiment, a hydrogel composition exhibits an elastic modulus of, e.g., at most 25 Pa, at most 50 Pa, at most 75 Pa, at most 100 Pa, at most 125 Pa, at most 150 Pa, at most 175 Pa, at most 200 Pa, at most 250 Pa, at most 300 Pa, at most 350 Pa, at most 400 Pa, at most 450 Pa, at most 500 Pa, at most 550 Pa, at most 600 Pa, at most 650 Pa, at most 700 Pa, at most 750 Pa, at most 800 Pa, at most 850 Pa, at most 900 Pa, at most 950 Pa, at most 1,000 Pa, at most 1,200 Pa, at most 1,300 Pa, at most 1,400 Pa, at most 1,500 Pa, or at most 1,600 Pa. In still other aspects of this embodiment, a hydrogel composition exhibits an elastic modulus of, e.g., about 25 Pa to about 150 Pa, about 25 Pa to about 300 Pa, about 25 Pa to about 500 Pa, about 25 Pa to about 800 Pa, about 125 Pa to about 300 Pa, about 125 Pa to about 500 Pa, about 125 Pa to about 800 Pa, about 400 to about 1,600 Pa, about 500 Pa to about 1,600 Pa, about 600 Pa to about 1,600 Pa, about 700 Pa to about 1,600 Pa, about 800 Pa to about 1,600 Pa, about 900 Pa to about 1,600 Pa, about 1,000 Pa to about 1,600 Pa, about 1,100 Pa to about 1,600 Pa, about 1,200 Pa to about 1,600 Pa, about 500 Pa to about 2,500 Pa, about 1,000 Pa to about 2,500 Pa, about 1,500 Pa to about 2,500 Pa, about 2,000 Pa to about 2,500 Pa, about 1,300 Pa to about 1,600 Pa, about 1,400 Pa to about 1,700 Pa, about 1,500 Pa to about 1,800 Pa, about 1,600 Pa to about 1,900 Pa, about 1,700 Pa to about 2,000 Pa, about 1,800 Pa to about 2,100 Pa, about 1,900 Pa to about 2,200 Pa, about 2,000 Pa to about 2,300 Pa, about 2,100 Pa to about 2,400 Pa, or about 2,200 Pa to about 2,500 Pa. In still other aspects, there is provided a hydrogel composition exhibiting an elastic modulus of, e.g., about 50 Pa to about 5,000 Pa, about 100 Pa to about 5,000 Pa, about 100 to about 4,000 Pa, about 100 to about 3,000 Pa, about 100 to about 2,000 Pa, or about 100 to about 1,000 Pa, or about 500 Pa. In still other aspects, there is provided a hydrogel composition exhibiting an elastic modulus of, e.g., about 20 Pa to about 3,000 Pa, about 20 Pa to about 2,000 Pa, about 20 to about 1,000 Pa, about 50 to about 3,000 Pa, about 50 to about 2,000 Pa, or about 50 to about 1,000 Pa.

In a further embodiment, a hydrogel composition disclosed herein exhibits a high elastic modulus (high storage modulus (G')) of, e.g., at least about 500 Pa.

In another embodiment, a coacervate HA hydrogel composition disclosed herein exhibits a viscous modulus. In aspects of this embodiment, a hydrogel composition exhibits a viscous modulus of, e.g., about 10 Pa, about 20 Pa, about 30 Pa, about 40 Pa, about 50 Pa, about 60 Pa, about 70 Pa, about 80 Pa, about 90 Pa, about 100 Pa, about 150 Pa, about 200 Pa, about 250 Pa, about 300 Pa, about 350 Pa, about 400 Pa, about 450 Pa, about 500 Pa, about 550 Pa, about 600 Pa, about 650 Pa, or about 700 Pa. In other aspects of this embodiment, a hydrogel composition exhibits a viscous modulus of, e.g., at most 10 Pa, at most 20 Pa, at most 30 Pa, at most 40 Pa, at most 50 Pa, at most 60 Pa, at most 70 Pa, at most 80 Pa, at most 90 Pa, at most 100 Pa, at most 150 Pa, at most 200 Pa, at most 250 Pa, at most 300 Pa, at most 350 Pa, at most 400 Pa, at most 450 Pa, at most 500 Pa, at most 550 Pa, at most 600 Pa, at most 650 Pa, or at most 700 Pa. In yet other aspects of this embodiment, a hydrogel composition exhibits a viscous modulus of, e.g., about 10 Pa to about 30 Pa, about 10 Pa to about 50 Pa, about 10 Pa to about 100 Pa, about 10 Pa to about 150 Pa, about 70 Pa to about 100 Pa, about 50 Pa to about 350 Pa, about 150 Pa to about 450 Pa, about 250 Pa to about 550 Pa, about 350 Pa to about 700 Pa, about 50 Pa to about 150 Pa, about 100 Pa to about 200 Pa, about 150 Pa to about 250 Pa, about 200 Pa to about 300 Pa, about 250 Pa to about 350 Pa, about 300 Pa to about 400 Pa, about 350 Pa to about 450 Pa, about 400 Pa to about 500 Pa, about 450 Pa to about 550 Pa, about 500 Pa to about 600 Pa, about 550 Pa to about 650 Pa, or about 600 Pa to about 700 Pa.

In another embodiment, a coacervate HA hydrogel composition disclosed herein exhibits a tan δ. In aspects of this embodiment, a hydrogel composition exhibits a tan δ of, e.g., about 0.1, about 0.2, about 0.3, about 0.4, about 0.5, about 0.6, about 0.7, about 0.8, about 0.9, about 1.0, about 1.1, about 1.2, about 1.3, about 1.4, about 1.5, about 1.6, about 1.7, about 1.8, about 1.9, about 2.0, about 2.1, about 2.2, about 2.3, about 2.4, or about 2.5. In other aspects of this embodiment, a hydrogel composition exhibits a tan δ of, e.g., at most 0.1, at most 0.2, at most 0.3, at most 0.4, at most 0.5, at most 0.6, at most 0.7, at most 0.8, at most 0.9, at most 1.0, at most 1.1, at most 1.2, at most 1.3, at most 1.4, at most 1.5, at most 1.6, at most 1.7, at most 1.8, at most 1.9, at most 2.0, at most 2.1, at most 2.2, at most 2.3, at most 2.4, or at most 2.5. In yet other aspects of this embodiment, a hydrogel composition exhibits a tan δ of, e.g., about 0.1 to about 0.3, about 0.3 to about 0.5, about 0.5 to about 0.8, about 1.1 to about 1.4, about 1.4 to about 1.7, about 0.3 to about 0.6, about 0.1 to about 0.5, about 0.5 to about 0.9, about 0.1 to about 0.6, about 0.1 to about 1.0, about 0.5 to about 1.5, about 1.0 to about 2.0, or about 1.5 to about 2.5.

A coacervate HA hydrogel composition disclosed herein may be further processed by pulverizing the hydrogel into particles and optionally mixed with a carrier phase, such as a pharmaceutically acceptable carrier such as, e.g., water, a saline solution (including PBS), glycerol and/or non-crosslinked HA, to form an injectable or topical substance like a solution, oil, lotion, gel, ointment, cream, slurry, salve, or paste. As such, the disclosed coacervate HA hydrogel compositions may be monophasic or multiphasic compositions. A coacevate HA hydrogel may be milled to a particle size from about 10 µm to about 1000 µm in diameter, such as about 15 µm to about 30 µm, about 50 µm to about 75 µm, about 100 µm to about 150 µm, about 200 µm to about 300 µm, about 450 µm to about 550 µm, about 600 µm to about 700 µm, about 750 µm to about 850 µm, or about 900 µm to about 1,000 µm. The particles may be further processed by homogenization or sizing through a fine porous screen.

Aspects of the present specification provide, in part, a composition disclosed herein that is injectable. As used herein, the term "injectable" refers to a material having the properties necessary to administer the composition into an individual's skin or other soft tissue using an injection device with a needle, such as a fine needle. As used herein, the term "fine needle" refers to a needle that is 27 gauge or higher (wherein a higher gauge indicates a smaller outer diameter). Injectability of a composition disclosed herein can be accomplished by sizing the hydrogel particles as discussed above. In some embodiments, injectability of a composition disclosed herein can be accomplished without further processing of the hydrogel by homogenization or sizing through a fine porous screen.

In aspect of this embodiment, a coacervate HA hydrogel composition disclosed herein is injectable through a fine needle. In other aspects of this embodiment, a hydrogel composition disclosed herein is injectable through a needle of, e.g., 18 gauge, 19 gauge, 20 gauge, 21 gauge, 22 gauge, 23 gauge, 24 gauge, 25 gauge, 26 gauge, 27 gauge, 28 gauge, 29 gauge, 30 gauge, 31 gauge or 32 gauge. In yet other aspects of this embodiment, a hydrogel composition disclosed herein is injectable through a needle of, e.g., at least 21 gauge, at least 25 gauge, at least 27 gauge, at least 30 gauge, or at least 32 gauge. In still other aspects of this embodiment, a hydrogel composition disclosed herein is injectable through a needle of, e.g., 18 gauge to 35 gauge, 18 gauge to 25 gauge, 21 gauge to 35 gauge, 21 gauge to 34 gauge, 21 gauge to 33 gauge, 21 gauge to 32 gauge, 21 gauge to 27 gauge, or 27 gauge to 32 gauge.

In aspects of this embodiment, a coacervate HA hydrogel composition disclosed herein can be injected with an extrusion force of about 60 N, about 55 N, about 50 N, about 45 N, about 40 N, about 35 N, about 30 N, about 25 N, about 20 N, or about 15 N at speeds of 100 mm/min. In other aspects of this embodiment, a hydrogel composition disclosed herein can be injected through a 27 gauge needle with an extrusion force of about 60 N or less, about 55 N or less, about 50 N or less, about 45 N or less, about 40 N or less, about 35 N or less, about 30 N or less, about 25 N or less, about 20 N or less, about 15 N or less, about 10 N or less, or about 5 N or less. In yet other aspects of this embodiment, a hydrogel composition disclosed herein can be injected through a 30 gauge needle with an extrusion force of about 60 N or less, about 55 N or less, about 50 N or less, about 45 N or less, about 40 N or less, about 35 N or less, about 30 N or less, about 25 N or less, about 20 N or less, about 15 N or less, about 10 N or less, or about 5 N or less. In still other aspects of this embodiment, a hydrogel composition disclosed herein can be injected through a 32 gauge needle with an extrusion force of about 60 N or less, about 55 N or less, about 50 N or less, about 45 N or less, about 40 N or less, about 35 N or less, about 30 N or less, about 25 N or less, about 20 N or less, about 15 N or less, about 10 N or less, or about 5 N or less.

Aspects of the present invention provide, in part, a hydrogel composition disclosed herein that exhibits cohesivity. Cohesivity, also referred to as cohesive attraction, cohesive force, or compression force is a physical property of a material, caused by the intermolecular attraction between like-molecules within the material that acts to unite the molecules. Cohesivity is expressed in terms of grams-force (gmf). Cohesiveness is affected by, among other factors, the molecular weight ratio of the initial free HA polysaccharide, the degree of crosslinking of HA polysaccharides, the amount of residual free HA polysaccharides following crosslinking, and the pH of the hydrogel composition. A composition should be sufficiently cohesive as to remain localized to a site of administration. Additionally, in certain applications, a sufficient cohesiveness is important for a composition to retain its shape, and thus functionality, in the event of mechanical load cycling. As such, in one embodiment, a coacervate HA hydrogel composition disclosed herein exhibits cohesivity, on par with water. In yet another embodiment, a hydrogel composition disclosed herein exhibits sufficient cohesivity to remain localized to a site of administration. In still another embodiment, a hydrogel composition disclosed herein exhibits sufficient cohesivity to retain its shape. In a further embodiment, a hydrogel composition disclosed herein exhibits sufficient cohesivity to retain its shape and functionality.

Aspects of the present specification provide, in part, a coacervate HA hydrogel composition disclosed herein that exhibits a physiologically-acceptable osmolarity. As used herein, the term "osmolarity" refers to the concentration of osmotically active solutes in solution. As used herein, the term "a physiologically-acceptable osmolarity" refers to an osmolarity in accord with, or characteristic of, the normal functioning of a living organism. As such, administration of a hydrogel composition as disclosed herein exhibits an osmolarity that has substantially no long term or permanent detrimental effect when administered to a mammal. Osmolarity is expressed in terms of osmoles of osmotically active solute per liter of solvent (Osmol/L or Osm/L). Osmolarity is distinct from molarity because it measures moles of osmotically active solute particles rather than moles of solute. The distinction arises because some compounds can dissociate in solution, whereas others cannot. The osmolarity of a solution can be calculated from the following expression: Osmol/L = ∑ϕi ηi Ci, where ϕ is the osmotic coefficient, which accounts for the degree of non-ideality of the solution; η is the number of particles (e.g. ions) into which a molecule dissociates; and C is the molar concentration of the solute; and i is the index representing the identity of a particular solute. The osmolarity of a hydrogel composition disclosed herein can be measured using a conventional method that measures solutions.

In an embodiment, a coacervate HA hydrogel hydrogel composition disclosed herein exhibits a physiologically-acceptable osmolarity. In aspects of this embodiment, a hydrogel composition exhibits an osmolarity of, e.g., about 100 mOsm/L, about 150 mOsm/L, about 200 mOsm/L, about 250 mOsm/L, about 300 mOsm/L, about 350 mOsm/L, about 400 mOsm/L, about 450 mOsm/L, or about 500 mOsm/L. In other aspects of this embodiment, a hydrogel composition exhibits an osmolarity of, e.g., at least 100 mOsm/L, at least 150 mOsm/L, at least 200 mOsm/L, at least 250 mOsm/L, at least 300 mOsm/L, at least 350 mOsm/L, at least 400 mOsm/L, at least 450 mOsm/L, or at least 500 mOsm/L. In yet other aspects of this embodiment, a hydrogel composition exhibits an osmolarity of, e.g., at most 100 mOsm/L, at most 150 mOsm/L, at most 200 mOsm/L, at most 250 mOsm/L, at most 300 mOsm/L, at most 350 mOsm/L, at most 400 mOsm/L, at most 450 mOsm/L, or at most 500 mOsm/L. In still other aspects of this embodiment, a hydrogel composition exhibits an osmolarity of, e.g., about 100 mOsm/L to about 500 mOsm/L, about 200 mOsm/L to about 500 mOsm/L, about 200 mOsm/L to about 400 mOsm/L, about 300 mOsm/L to about 400 mOsm/L, about 270 mOsm/L to about 390 mOsm/L, about 225 mOsm/L to about 350 mOsm/L, about 250 mOsm/L to about 325 mOsm/L, about 275 mOsm/L to about 300 mOsm/L, or about 285 mOsm/L to about 290 mOsm/L.

Aspects of the present invention provide, in part, a coacervate HA hydrogel composition disclosed herein that exhibits substantial stability. As used herein, the term "stability" or "stable" when referring to a hydrogel composition disclosed herein refers to a composition that is not prone to degrading, decomposing, or breaking down to any substantial or significant degree while stored before administration to an individual. As used herein, the term "substantial heat stability", "substantially heat stable", "autoclave stable", or "steam sterilization stable" refers to a hydrogel composition disclosed herein that is substantially stable when subjected to a heat treatment as disclosed herein.

Stability of a hydrogel composition disclosed herein (including hydrogel compositions further comprising any agent or additive disclosed herein) can be determined by subjecting a hydrogel composition to a heat treatment, such as, e.g., steam sterilization at normal pressure or under pressure (e.g., autoclaving). Preferably the heat treatment is carried out at a temperature of at least about 100 °C for between about one minute and about 10 minutes. Substantial stability of a hydrogel composition disclosed herein can be evaluated 1) by determining the change in the extrusion force (ΔF) of a hydrogel composition disclosed herein after sterilization, where the change in extrusion force of less than 2N is indicative of a substantially stable hydrogel composition as measured by (the extrusion force of a hydrogel composition with the specified agents and/or additives) minus (the extrusion force of the a hydrogel composition without the added agents and/or additives); and/or 2) by determining the change in rheological properties of a hydrogel composition disclosed herein after sterilization, where the change in tan δ 1 Hz of less than 0.1 is indicative of a substantially stable hydrogel composition as measured by (tan δ 1 Hz of gel formulation with agents and/or additives) minus (tan δ 1 Hz of gel formulation without agents and/or additives). As such, a substantially stable hydrogel composition disclosed herein retains one or more of the following characteristics after sterilization: homogeneousness, extrusion force, cohesiveness, anionic HA concentration, cationic polysaccharide concentration, agent or additive concentration, osmolarity, pH, or other rheological characteristics desired by the hydrogel before the heat treatment.

In an embodiment, a coacervate HA hydrogel disclosed herein is processed using a heat treatment that maintains the desired hydrogel properties disclosed herein. In aspects of this embodiment, a coacervate HA hydrogel disclosed herein is processed using a heat treatment of, e.g., about 100 °C, about 105 °C, about 110 °C, about 115 °C, about 120 °C, about 125 °C, or about 130 °C. In other aspects of this embodiment, a coacervate HA hydrogel disclosed herein is processed using a heat treatment of, e.g., at least 100 °C, at least 105 °C, at least 110 °C, at least 115 °C, at least 120 °C, at least 125 °C, or at least 130 °C. In yet other aspects of this embodiment, a coacervate HA hydrogel disclosed herein is processed using a heat treatment of, e.g., about 100 °C to about 120 °C, about 100 °C to about 125 °C, about 100 °C to about 130 °C, about 100 °C to about 135 °C, about 110 °C to about 120 °C, about 110 °C to about 125 °C, about 110 °C to about 130 °C, about 110 °C to about 135 °C, about 120 °C to about 125 °C, about 120 °C to about 130 °C, about 120 °C to about 135 °C, about 125 °C to about 130 °C, or about 125 °C to about 135 °C.

Long term stability of a hydrogel composition disclosed herein can be determined by subjecting a coacervate HA hydrogel to a heat treatment, such as, e.g., storage in an about 45 °C environment for about 60 days. Long term stability of a coacervate HA hydrogel disclosed herein can be evaluated 1) by assessing the clarity and color of a hydrogel composition after the 45 °C heat treatment, with a clear and uncolored hydrogel composition being indicative of a substantially stable hydrogel composition; 2) by determining the change in the extrusion force (ΔF) of a coacervate HA hydrogel disclosed herein after the 45 °C heat treatment, where the change in extrusion force less 2N is indicative of a substantially stable coacervate HA hydrogel composition as measured by (the extrusion force of a hydrogel composition before the 45 °C heat treatment) minus (the extrusion force of the a hydrogel composition after the 45 °C heat treatment); and/or 3) by determining the change in rheological properties of a coacervate HA hydrogel disclosed herein after sterilization, where the change in tan δ 1 Hz of less than 0.1 is indicative of a substantially stable coacervate HA hydrogel composition as measured by (tan δ 1 Hz of gel formulation before the 45 °C heat treatment) minus (tan δ 1 Hz of gel formulation after the 45 °C heat treatment). As such, a long term stability of a coacervate HA hydrogel composition disclosed herein is evaluated by retention of one or more of the following characteristics after the 45 °C heat treatment: clarity (transparency and translucency), homogeneousness, and cohesiveness.

In aspects of this embodiment, a coacervate HA hydrogel composition is substantially stable at room temperature for, e.g., about 3 months, about 6 months, about 9 months, about 12 months, about 15 months, about 18 months, about 21 months, about 24 months, about 27 months, about 30 months, about 33 months, or about 36 months. In other aspects of this embodiment, a coacervate HA hydrogel composition is substantially stable at room temperature for, e.g., at least 3 months, at least 6 months, at least 9 months, at least 12 months, at least 15 months, at least 18 months, at least 21 months, at least 24 months, at least 27 months, at least 30 months, at least 33 months, or at least 36 months. In other aspects of this embodiment, a coacervate HA hydrogel composition is substantially stable at room temperature for, e.g., about 3 months to about 12 months, about 3 months to about 18 months, about 3 months to about 24 months, about 3 months to about 30 months, about 3 months to about 36 months, about 6 months to about 12 months, about 6 months to about 18 months, about 6 months to about 24 months, about 6 months to about 30 months, about 6 months to about 36 months, about 9 months to about 12 months, about 9 months to about 18 months, about 9 months to about 24 months, about 9 months to about 30 months, about 9 months to about 36 months, about 12 months to about 18 months, about 12 months to about 24 months, about 12 months to about 30 months, about 12 months to about 36 months, about 18 months to about 24 months, about 18 months to about 30 months, or about 18 months to about 36 months.

Aspects of the present specification provide, in part, a coacervate HA hydrogel composition disclosed herein that is a pharmaceutically-acceptable composition. As used herein, the term "pharmaceutically acceptable" means any molecular entity or composition that does not produce an adverse, allergic or other untoward or unwanted reaction when administered to an individual. A pharmaceutically-acceptable coacervate HA hydrogel composition is useful for medical and veterinary applications. A pharmaceutically-acceptable coacervate HA hydrogel composition may be administered to an individual alone, or in combination with other supplementary active ingredients, agents, drugs or hormones. As used herein, "pharmaceutically acceptable" and "physiologically acceptable" may be used interchangeably.

Aspects of the present specification provide, in part, a coacervate HA hydrogel composition as disclosed herein comprising a pharmacologically acceptable excipient. As used herein, the term "pharmacologically acceptable excipient" is synonymous with "pharmacological excipient" or "excipient" and refers to any excipient that has substantially no long term or permanent detrimental effect when administered to mammal and encompasses compounds such as, e.g., a stabilizing agent, a bulking agent, a cryo-protectant, a lyo-protectant, an additive, a vehicle, a carrier, a diluent, or an auxiliary. An excipient generally is mixed with an active ingredient, or permitted to dilute or enclose the active ingredient and can be a solid, semi-solid, or liquid agent. It is also envisioned that a pharmaceutical composition as disclosed herein can include one or more pharmaceutically acceptable excipients that facilitate processing of an active ingredient into pharmaceutically acceptable compositions. Insofar as any pharmacologically acceptable excipient is not incompatible with the active ingredient, its use in pharmaceutically acceptable compositions is contemplated. Non-limiting examples of pharmacologically acceptable excipients (including carriers) can be found in, e.g., Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003).

As used herein, "carrier," and "acceptable carrier" may be used interchangeably and refer to a carrier which may be combined with the presently disclosed hydrogel in order to provide a desired composition. Those of ordinary skill in the art will recognize a number of carriers that are well known for making specific cosmetic compositions. Physiologically and/or pharmaceutically acceptable carriers include, without limitation, saline solutions, such as phosphate buffer saline (PBS), glycerol and non-crosslinked HA.

It is further envisioned that a coacervate HA hydrogel composition disclosed herein may optionally include, without limitation, other pharmaceutically acceptable components, including, without limitation, buffers, carriers, preservatives, tonicity adjusters, salts, antioxidants, osmolality adjusting agents, emulsifying agents, wetting agents, and the like.

A pharmaceutically acceptable buffer is a buffer that can be used to prepare a hydrogel composition disclosed herein, provided that the resulting preparation is pharmaceutically acceptable. Non-limiting examples of pharmaceutically acceptable buffers include acetate buffers, borate buffers, citrate buffers, neutral buffered salines, phosphate buffers, and phosphate buffered salines. Non-limiting examples of concentrations of physiologically-acceptable buffers occur within the range of about 0.1 mM to about 900 mM. The pH of pharmaceutically acceptable buffers may be adjusted, provided that the resulting preparation is pharmaceutically acceptable. It is understood that acids or bases can be used to adjust the pH of a pharmaceutical composition as needed. Non-limiting examples of physiologically-acceptable pH occur within the range of about pH 5.0 to about pH 8.5. For example, the pH of a hydrogel composition disclosed herein can be about 5.0 to about 8.0, or about 6.5 to about 7.5, about 7.0 to about 7.4, or about 7.1 to about 7.3.

Pharmaceutically acceptable preservatives include, without limitation, sodium metabisulfite, sodium thiosulfate, acetylcysteine, butylated hydroxyanisole and butylated hydroxytoluene. Pharmaceutically acceptable preservatives include, without limitation, benzalkonium chloride, chlorobutanol, thimerosal, phenylmercuric acetate, phenylmercuric nitrate, a stabilized oxy chloro composition, such as, e.g., PURITE® (Allergan, Inc. Irvine, CA) and chelants, such as, e.g., DTPA or DTPA-bisamide, calcium DTPA, and CaNaDTPA-bisamide.

Pharmaceutically acceptable tonicity adjustors useful in a coacervate HA hydrogel composition disclosed herein include, without limitation, salts such as, e.g., sodium chloride and potassium chloride; and glycerin. The composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. It is understood that these and other substances known in the art of pharmacology can be included in a pharmaceutical composition disclosed herein. Other non-limiting examples of pharmacologically acceptable components can be found in, e.g., Ansel, supra, (1999); Gennaro, supra, (2000); Hardman, supra, (2001); and Rowe, supra, (2003).

The invention further provides general methods of preparing coacervate HA hydrogels. In some embodiments, the methods comprise forming a complex comprising ion bonding interactions between anionic HA ions and cationic polysaccharide ions.

The invention also provides for methods of preparing coacervate HA hydrogels with different rheological profiles, which may be formed based on the number, identity, and/or pKa value(s) of the anions and/or cations of each of anionic HA and the cationic polysaccharides, respectively.

In some embodiments, the hydrogels are formed through ionic interactions between non-crosslinked anionic HA and cationic polysaccharides, the anionic HA having anions including, but not limited to, carboxylate, sulfonate, and/or phosphonate; the cationic polysaccharides having cations including, but not limited to, primary ammonium, quaternary ammonium and/or guanidinium.

In other embodiments, the hydrogels are formed through ionic interactions between crosslinked anionic HA and cationic polysaccharides, the anionic HA having anions including, but not limited to, carboxylate, sulfonate, and/or phosphonate; the cationic polysaccharides having cations including, but not limited to, primary ammonium, quaternary ammonium and/or guanidinium. In some embodiments, the cationic polysaccharide serves as a cohesive agent to improve crosslinked anionic HA gel cohesivity.

In some embodiments, the hydrogels are prepared by forming an ionic complex between a homoanionic HA and a homocationic polysaccharide. In some embodiments, the homoanionic HA is HA. In some embodiments, the homocationic polysaccharide is a cationic HA. In some embodiments, the homocationic polysaccharide is chitosan. In further embodiments, the homocationic polysaccharide is trimethyl chitosan.

In some embodiments, the hydrogels are prepared by forming an ionic complex between a homoanionic HA and a heterocationic polysaccharide. In some embodiments, the homoanionic HA is HA.

In some embodiments, the hydrogels are prepared by forming an ionic complex between a heteroanionic HA and a homocationic polysaccharide. In some embodiments, the homocationic HA is a cationic HA. In other embodiments, the homocationic polysaccharide is chitosan. In further embodiments, the homocationic polysaccharide is trimethyl chitosan.

In some embodiments, the hydrogels are prepared by forming an ionic complex between a heteroanionic HA and a heterocationic polysaccharide.

Non-limiting embodiments of the method of forming hydrogels of the invention include forming an ionic complex between an anionic polysaccharide and a cationic polysaccharide, wherein the anionic polysaccharide is selected from the anionic polysaccharide embodiments of Table 1, and the cationic polysaccharide is selected from the cationic polysaccharide embodiments of Table 1, without limitation with respect to the combination(s) of anionic and cationic polysaccharides.

In one embodiment, there is provided a method of preparing a hydrogel comprising non-crosslinked HA and non-crosslinked cationic HA, wherein the method comprises separately hydrating each polysaccharide, for example, by dissolving each in phosphate buffered saline; followed by mixing of the hydrated HA with the hydrated cationic HA at a variable charge ratio, for example, from about 1:20 to about 20:1, until a coacervate gel is achieved. Optionally, centrifigation is applied to remove excessive water.

In another embodiment, there is provided a method of preparing a hydrogel comprising crosslinked HA and non-crosslinked cationic polysaccharide, such as non-crosslinked cationic HA containing guanidinium cationic groups, the method comprising mixing the crosslinked HA with 1 wt% non-crosslinked cationic HA containing guanidinium cationic groups in a buffer (e.g., pH 7.5 PBS buffer) at a variable charge ratio (-COOH/guanidinium) of about 1:10 to about 50:1 until the coacervate gel is achieved.

In another embodiment, there is provided a method of preparing a hydrogel comprising crosslinked HA and chitosan, the method comprising providing hydrated crosslinked HA (i.e., an HA gel, such as JUP), providing highly pure chitosan (HPC), dissolving the HPC in acidic aqueous solution, optionally steam sterilizing the HPC in the acidic aqueous solution, diluting the resulting solution with water (e.g., to achieve an HPC concentration ranging from 0.4 mg/ml to 1.9 mg/ml), and mixing the dilute HPC solution with hydrated crosslinked HA to achieve the coacervate hydrogel comprising crosslinked HA and chitosan.

In other aspects, a hydrogel of the present invention is prepared by combining about 0.01 to about 1 molar equivalent of cationic polysaccharide with about 1 molar equivalent of the anionic polysaccharide. In further aspects, about 0.02 to about 0.5 molar equivalent of cationic polysaccharide is combined with 1 molar equivalent of the anionic polysaccharide. In yet further aspects, about 0.04 to about 0.2 molar equivalent of cationic polysaccharide is combined with about 1 molar equivalent of the anionic polysaccharide. Preferably, the anionic polysaccharide is HA. More preferably, the anionic polysaccharide is crosslinked HA, and the cationic polysaccharide is chitosan.

Aspects of the present specification provide, in part, a method of treating a soft tissue condition of an individual by administering a dermal filler composition disclosed herein.

As used herein, "soft tissues" refers to tissues that surround, support, or connect other structures or organs of the body. Non-limiting examples of soft tissues include connective tissues, such as skin, fibrous tissues, fat, fascia, tendons, ligaments, and synovial membranes. Soft tissues may also include non-connective tissues, such as nerves, muscles and blood vessels.

As used herein, the term "treating" refers to augmenting a soft tissue, improving soft tissue quality, or reducing a soft tissue defect; or reducing or eliminating in an individual a cosmetic symptom of a soft tissue condition characterized by a soft tissue imperfection and/or defect; or delaying or preventing in an individual the onset of a cosmetic symptom of a condition characterized by a soft tissue imperfection and/or defect. For example, the term "treating" can mean reducing a symptom of a condition characterized by a soft tissue defect by, e.g., at least 20%, at least 30%, at least 40%, at least 50%, at least 60%, at least 70%, at least 80%, at least 90% or at least 100%. The effectiveness of a dermal filler composition disclosed herein in treating a condition characterized by a soft tissue quality and/or defect can be determined by observing one or more cosmeticand/or physiological indicators associated with the condition. The effectiveness of a soft tissue augmentation or the improvement in soft tissue quality and/or defect disorder also can be indicated by a reduced need for a concurrent therapy. Those of skill in the art will know the appropriate symptoms or indicators associated with specific soft tissue defect and will know how to determine if an individual is a candidate for treatment with a composition disclosed herein.

In certain aspects, the method comprises administering a dermal filler of the invention comprising a coacervate HA hydrogel into a soft tissue or skin of the subject at a depth of no greater than about 1 mm. In some embodiments, the composition is injected superficially, that is, at a depth of a depth of no greater than about 0.8 mm, no greater than about 0.6 mm, or no greater than about 0.4 mm.

In another aspect, the present invention provides for methods of improving the aesthetic appearance of a subject, including the face of a subject. The present methods generally comprise the steps of administering a dermal filler comprising a coacervate HA hydrogel into a soft tissue or skin of the subject, such as the subject's face. In certain aspects, the method generally comprises administering a dermal filler of the invention comprising a coacervate HA hydrogel into a skin region, such as a dermal region, of the subject, such as the subject's face, at a depth of no greater than about 1 mm. In some embodiments, the composition is injected superficially, that is, at a depth of a depth of no greater than about 0.8 mm, no greater than about 0.6 mm, or no greater than about 0.4 mm.

A hydrogel composition is administered to an individual. An individual, also referred to as a subject or patient herein, is typically a human being of any age, gender or race. Typically, any individual who is a candidate for a conventional procedure to treat a soft tissue condition is a candidate for a method disclosed herein. Although a subject experiencing the signs of aging skin is an adult, subjects experiencing premature aging or other skin conditions suitable for treatment (for example, a scar) can also be treated with a hydrogel composition disclosed herein. In addition, the presently disclosed hydrogel compositions and methods may apply to individuals seeking a small/moderate enlargement, shape change or contour alteration of a body part or region, which may not be technically possible or aesthetically acceptable with existing soft tissue implant technology. Pre-operative evaluation typically includes routine history and physical examination in addition to thorough informed consent disclosing all relevant risks and benefits of the procedure.

The hydrogel composition and methods disclosed herein are useful in treating a soft tissue condition. A soft tissue condition includes, without limitation, a soft tissue imperfection and/or defect. Non-limiting examples of a soft tissue condition include a facial imperfection and/or defect, such as, e.g., a facial augmentation, a facial reconstruction, a mesotherapy, Parry-Romberg syndrome, lupus erythematosus profundus, dermal divots, scars, sunken cheeks, thin lips, nasal imperfections or defects, retro-orbital imperfections or defects, a facial fold, line and/or wrinkle like a glabellar line, a nasolabial line, a perioral line, and/or a marionette line, and/or other contour deformities or imperfections of the face; a neck imperfection and/or defect; a skin imperfection and/or defect; other soft tissue imperfections and/or defects, such as, e.g., an augmentation or a reconstruction of the upper arm, lower arm, hand, shoulder, back, torso including abdomen, buttocks, upper leg, lower leg including calves, foot including plantar fat pad, eye, genitals, or other body part, region or area. As used herein, the term "mesotherapy" refers to a non-surgical cosmetic treatment technique of the skin involving intra-epidermal, intra-dermal, and/or subcutaneous injection of an agent administered as small multiple droplets into the epidermis, dermo-epidermal junction, and/or the dermis.

The amount of a hydrogel composition used with any of the methods as disclosed herein will typically be determined based on the alteration and/or improvement desired, the reduction and/or elimination of a soft tissue condition symptom desired, the effect desired by the individual and/or physician, and the body part or region being treated. The effectiveness of composition administration may be manifested by one or more of the following measures: altered and/or improved soft tissue shape, altered and/or improved soft tissue size, altered and/or improved soft tissue contour, altered and/or improved tissue function, improved patient satisfaction and/or quality of life, and decreased use of implantable foreign material.

As another example, effectiveness of the compositions and methods in treating a facial soft tissue may be manifested by one or more of the following measures: increased size, shape, and/or contour of facial feature like increased size, shape, and/or contour of lip, cheek or eye region; altered size, shape, and/or contour of facial feature like altered size, shape, and/or contour of lip, cheek or eye region shape; reduction or elimination of a wrinkle, fold or line in the skin; resistance to a wrinkle, fold or line in the skin; rehydration of the skin; increased elasticity to the skin; reduction or elimination of skin roughness; increased and/or improved skin tautness; reduction or elimination of stretch lines or marks; increased and/or improved skin tone, shine, brightness and/or radiance; increased and/or improved skin color, reduction or elimination of skin paleness; improved patient satisfaction and/or quality of life.

In aspects of this embodiment, the amount of a hydrogel composition administered is, e.g., about 0.01 g, about 0.05 g, about 0.1 g, about 0.5 g, about 1 g, about 5 g, about 10 g, about 20 g, about 30 g, about 40 g, about 50 g, about 60 g, about 70 g, about 80 g, about 90 g, about 100 g, about 150 g, or about 200 g. In other aspects of this embodiment, the amount of a hydrogel composition administered is, e.g., about 0.01 g to about 0.1 g, about 0.1 g to about 1 g, about 1 g to about 10 g, about 10 g to about 100 g, or about 50 g to about 200 g. In yet other aspects of this embodiment, the amount of a hydrogel composition administered is, e.g., about 0.01 mL, about 0.05 mL, about 0.1 mL, about 0.5 mL, about 1 mL, about 5 mL, about 10 mL, about 15 mL, about 20 mL, about 30 mL, about 40 mL, about 50 mL, about 60 mL, about 70 g, about 80 mL, about 90 mL, about 100 mL, about 150 mL, or about 200 mL. In other aspects of this embodiment, the amount of a hydrogel composition administered is, e.g., about 0.01 mL to about 0.1 mL, about 0.1 mL to about 1 mL, about 1 mL to about 10 mL, about 1 mL to about 20 mL, about 10 mL to about 100 mL, or about 50 mL to about 200 mL.

The duration of treatment will typically be determined based on the effect desired by the individual and/or physician and the body part or region being treated. In aspects of this embodiment, administration of a hydrogel composition disclosed herein can treat a soft tissue condition for, e.g., about 6 months, about 7 months, about 8 months, about 9 months, about 10 months, about 11 months, about 12 months, about 13 months, about 14 months, about 15 months, about 18 months, or about 24 months. In other aspects of this embodiment, administration of a hydrogel composition disclosed herein can treat a soft tissue condition for, e.g., at least 6 months, at least 7 months, at least 8 months, at least 9 months, at least 10 months, at least 11 months, at least 12 months, at least 13 months, at least 14 months, at least 15 months, at least 18 months, or at least 24 months. In yet aspects of this embodiment, administration of a hydrogel composition disclosed herein can treat a soft tissue condition for, e.g., about 6 months to about 12 months, about 6 months to about 15 months, about 6 months to about 18 months, about 6 months to about 21 months, about 6 months to about 24 months, about 9 months to about 12 months, about 9 months to about 15 months, about 9 months to about 18 months, about 9 months to about 21 months, about 6 months to about 24 months, about 12 months to about 15 months, about 12 months to about 18 months, about 12 months to about 21 months, about 12 months to about 24 months, about 15 months to about 18 months, about 15 months to about 21 months, about 15 months to about 24 months, about 18 months to about 21 months, about 18 months to about 24 months, or about 21 months to about 24 months.

Aspects of the present specification provide, in part, administering a hydrogel composition disclosed herein. As used herein, the term "administering" means any delivery mechanism that provides a composition disclosed herein to an individual that potentially results in a beneficial result. The actual delivery mechanism used to administer a composition to an individual can be determined by a person of ordinary skill in the art by taking into account factors, including, without limitation, the type of condition, the location of the condition, the cause of the condition, the severity of the condition, the degree of benefit desired, the duration of benefit desired, the particular composition used, the rate of biodegradation of the particular composition used, the nature of the other agents included in the particular composition used, the particular route of administration, the particular characteristics, history and risk factors of the individual, such as, e.g., age, weight, general health and the like, or any combination thereof. In an aspect of this embodiment, a composition disclosed herein is administered to an individual's soft tissue or skin by injection.

The route of administration of a hydrogel composition to an individual patient will typically be determined based on the cosmetic effect desired by the individual and/or physician and the body part or region being treated. A composition disclosed herein may be administered by any means known to persons of ordinary skill in the art including, without limitation, syringe with needle, a pistol (for example, a hydropneumatic-compression pistol), or by direct surgical implantation. The hydrogel composition disclosed herein can be administered into a soft tissue or skin, such as, e.g., a dermal region, a hypodermal region, or an even deeper region. For example, a hydrogel composition disclosed herein can be injected utilizing needles or cannulas with a diameter of about 0.26 mm to about 0.4 mm and a length ranging from about 4 mm to about 14 mm. Alternately, the needles or cannulas can be 21 to 32 gauge and have a length of about 4 mm to about 70 mm. Other suitable needle gauges are disclosed herein. Preferably, the needle is a single-use needle. The needle can be combined with a syringe, catheter, and/or a pistol. Cannulas may be of varying size and include rigid or flexible versions.

In addition, a composition disclosed herein can be administered once, or over a plurality of times. Ultimately, the timing used will follow quality care standards. For example, a dermal filler composition disclosed herein can be administered once or over several sessions with the sessions spaced apart by a few days, or weeks. For instance, an individual can be administered a dermal filler composition disclosed herein every 1, 2, 3, 4, 5, 6, or 7 days or every 1, 2, 3, or 4 weeks. The administration of a dermal filler composition disclosed herein to an individual can be on a monthly or bi-monthly basis or administered every 3, 6, 9, or 12 months.

Aspects of the present specification relate, in part, to skin. The skin is composed of three primary layers: the epidermis, which provides waterproofing and serves as a barrier to infection; the dermis, which serves as a location for the appendages of skin; and the hypodermis (subcutaneous adipose layer). The epidermis contains no blood vessels, and is nourished by diffusion from the dermis. The main type of cells which make up the epidermis are keratinocytes, melanocytes, Langerhans cells and Merkels cells. The skin includes the "dermal region."

Aspects of the present specification provide, in part, a dermal region. As used herein, the term "dermal region" refers to the region of skin comprising the epidermal-dermal junction and the dermis. The epi-dermal junction provides epidermal-dermal adherence, mechanical support for the epidermis, and a barrier to the exchange of cells and some large molecules across the junction. The dermis includes the superficial dermis (papillary region) and the deep dermis (reticular region).

The dermis is the layer of skin beneath the epidermis that consists of connective tissue and cushions the body from stress and strain. The dermis is tightly connected to the epidermis by a basement membrane. It also harbors many mechanoreceptor/nerve endings that provide the sense of touch and heat. It contains the hair follicles, sweat glands, sebaceous glands, apocrine glands, lymphatic vessels and blood vessels. The blood vessels in the dermis provide nourishment and waste removal from its own cells as well as from the Stratum basale of the epidermis. The dermis is structurally divided into two areas: a superficial area adjacent to the epidermis, called the papillary region, and a deep thicker area known as the reticular region.

The papillary region is composed of loose areolar connective tissue. It is named for its fingerlike projections called papillae that extend toward the epidermis. The papillae provide the dermis with a "bumpy" surface that interdigitates with the epidermis, strengthening the connection between the two layers of skin. The reticular region lies deep in the papillary region and is usually much thicker. It is composed of dense irregular connective tissue, and receives its name from the dense concentration of collagenous, elastic, and reticular fibers that weave throughout it. These protein fibers give the dermis its properties of strength, extensibility, and elasticity. Also located within the reticular region are the roots of the hair, sebaceous glands, sweat glands, receptors, nails, and blood vessels. Tattoo ink is held in the dermis. Stretch marks from pregnancy are also located in the dermis.

The hypodermis lies below the dermis. Its purpose is to attach the dermal region of the skin to underlying bone and muscle as well as supplying it with blood vessels and nerves. It consists of loose connective tissue and elastin. The main cell types are fibroblasts, macrophages and adipocytes (the hypodermis contains 50% of body fat). Fat serves as padding and insulation for the body.

In an aspect of this embodiment, a hydrogel composition disclosed herein is administered to an individual's skin by injection into a dermal region or a hypodermal region. In aspects of this embodiment, a hydrogel composition disclosed herein is administered to a dermal region of an individual by injection into, e.g., an epidermal-dermal junction region, a papillary region, a reticular region, or any combination thereof.

Other aspects of the present specification disclose, in part, a method of treating a skin condition comprises the step of administering to an individual in need thereof a hydrogel composition disclosed herein, wherein the administration of the composition improves the skin condition, thereby treating the skin condition. In an aspect of this embodiment, a skin condition is a method of treating skin dehydration comprises the step of administering to an individual suffering from skin dehydration a hydrogel composition disclosed herein, wherein the administration of the composition rehydrates the skin, thereby treating skin dehydration. In another aspect of this embodiment, a method of treating a lack of skin elasticity comprises the step of administering to an individual suffering from a lack of skin elasticity a hydrogel composition disclosed herein, wherein the administration of the composition increases the elasticity of the skin, thereby treating a lack of skin elasticity. In yet another aspect of this embodiment, a method of treating skin roughness comprises the step of administering to an individual suffering from skin roughness a hydrogel composition disclosed herein, wherein the administration of the composition decreases skin roughness, thereby treating skin roughness. In still another aspect of this embodiment, a method of treating a lack of skin tautness comprises the step of administering to an individual suffering from a lack of skin tautness a hydrogel composition disclosed herein, wherein the administration of the composition makes the skin tauter, thereby treating a lack of skin tautness.

In a further aspect of this embodiment, a method of treating a skin stretch line or mark comprises the step of administering to an individual suffering from a skin stretch line or mark a hydrogel composition disclosed herein, wherein the administration of the composition reduces or eliminates the skin stretch line or mark, thereby treating a skin stretch line or mark. In another aspect of this embodiment, a method of treating skin paleness comprises the step of administering to an individual suffering from skin paleness a hydrogel composition disclosed herein, wherein the administration of the composition increases skin tone or radiance, thereby treating skin paleness. In another aspect of this embodiment, a method of treating skin wrinkles comprises the step of administering to an individual suffering from skin wrinkles a hydrogel composition disclosed herein, wherein the administration of the composition reduces or eliminates skin wrinkles, thereby treating skin wrinkles. In yet another aspect of this embodiment, a method of treating skin wrinkles comprises the step of administering to an individual a hydrogel composition disclosed herein, wherein the administration of the composition makes the skin resistant to skin wrinkles, thereby treating skin wrinkles. The present invention also provides methods of treating a condition, the method comprising administering a coacervate HA hydrogel of the invention. In one embodiment, there is provided a method of reducing the appearance of wrinkles. In another embodiment, there is provided a method of sculpting soft tissue features, including soft tissue facial features. In another embodiment, there is provided a method of applying mechanical pressure against a tissue adjacent to the hydrogel. In other embodiments, there is a provided a method of provide lifting capacity to tissues adjacent to the administered hydrogel.

This invention also provides for uses of coacervate HA hydrogels. Non-limiting uses of coacervate HA hydrogels of the invention include use as volumizers in skin, or space occupying agents which fill in the voids within or under the skin to reduce the appearance of wrinkles, or to sculpt particular soft tissue features, such as soft tissue facial features. In some embodiments, coacervate HA hydrogels of the invention are used to apply mechanical pressure against a tissue adjacent to the hydrogel. In other embodiments, the coacervate HA hydrogels of the invention provide lifting capacity to tissues adjacent to the administered hydrogel. In other embodiments, the coacervate HA hydrogels are used to deliver cosmetic or other agents to a tissue of a subject. Non-limiting examples of an agent delivered with the coacervate HA hydrogel include an antioxidant, an anti-itching agent, an anti-cellulite agent, an anti-scarring agent, an anti-inflammatory agent, an anesthetic agent, an anti-irritant agent, a desquamating agent, a tensioning agent, an anti-acne agent, a skin-lightening agent, a pigmentation agent, an anti-pigmentation agent, a moisturizing agent and/or a vitamin.

### EXAMPLES

### Example 1. Materials for making the present hydrogels useful as dermal fillers

A. Non-crosslinked HA. Non-crosslinked HA with a molecular weight of about 50K to about 3 million Dalton was purchased from HTL Biotechnology (France).
B. Crosslinked HA. Sources of crosslinked HA include commercially available HA hydrogels, such as Juvederm® brand dermal filler, (Allergan, Inc.), e.g. Juvederm® Ultra Plus dermal filler or Juvederm® Voluma dermal filler. A research grade crosslinked HA having a storage modulus (G') of about 500 to about 1,000 Pa can also be used. Methods of preparing crosslinked HA are described by Lebreton (US 2010/0226988, US 2008/0089918, US 2010/0028438 and US 2006/0194758; *supra),* and Njikang et al. (US 2013/0096081; *supra).*
C. Cationic HA. Cationic HA can be made according to the procedures described herein, for example, as depicted below.

Scheme 3. Introduction of ammonium cations into HA. HA is coupled with HMDA in the presence of EDC to provide a cationic HA having primary ammonium ions.

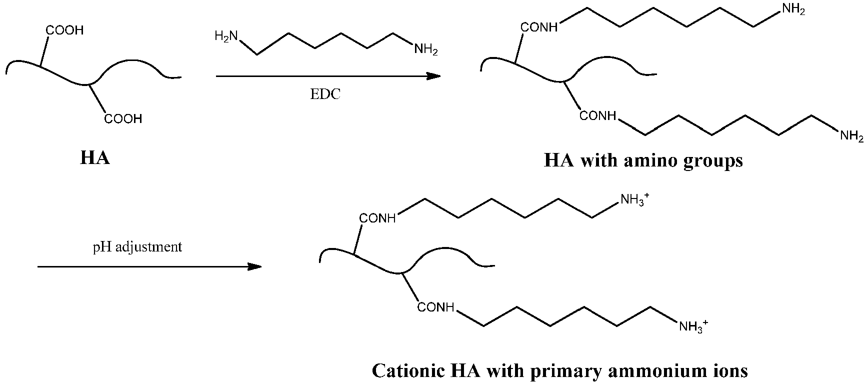

Scheme 4. Introduction of ammonium cations into HA. HA is coupled with Nₑₚₛᵢₗₒₙ-BOC-lysine-methyl ester in the presence of EDC, with subsequent removal of the BOC protecting group to provide a cationic HA having primary ammonium ions.

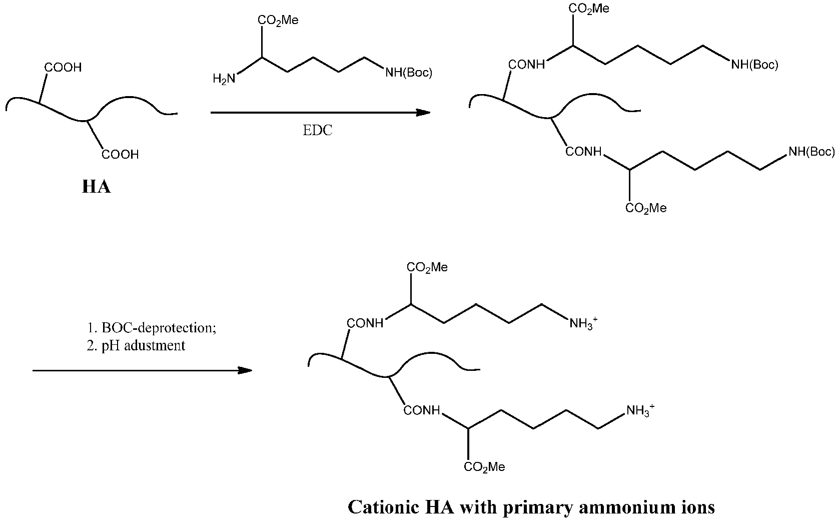

Scheme 5. Introduction of guanidinium cations into HA. Cationic HA prepared according to Scheme 3 is reacted with 1*H*-Pyrazole-1-carboxamidine to provide cationic HA having guanidinium ions.

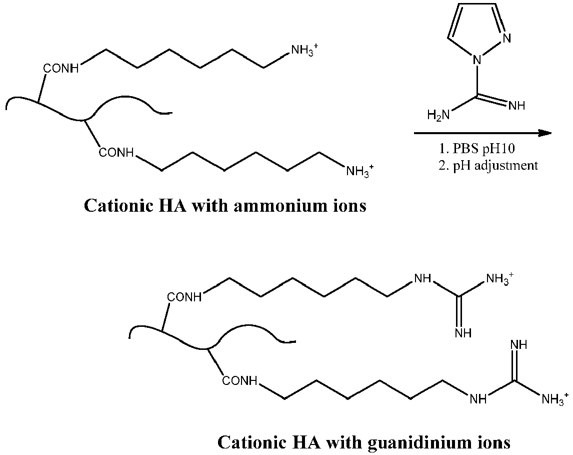

Scheme 6. Introduction of guanidinium cations into HA. Cationic HA prepared according to Scheme 4 is reacted with 1*H*-Pyrazole-1-carboxamidine to provide cationic HA having guanidinium ions. 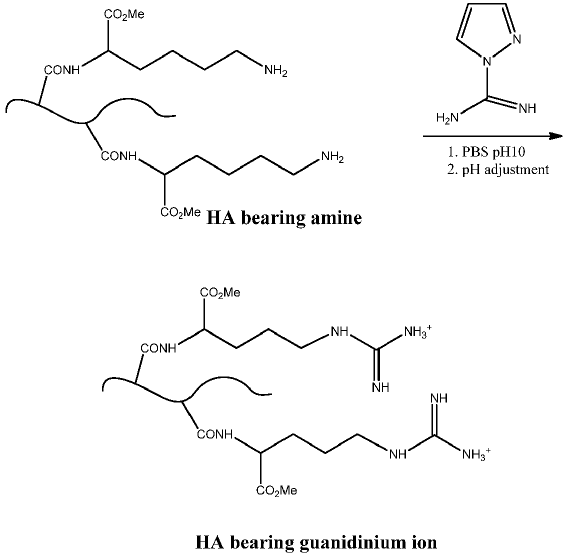
(i) Non-crosslinked cationic HA comprising primary ammonium ions. Briefly, in a 15 ml syringe, about 50 mg of non-crosslinked HA (MW: 300~800K dalton) and 14.4 mg of hexamethylenediamine (HMDA) will be dissolved in pH 5.0 MES buffer. Then, to a separate 15 mL syringe containing 5 ml of pH 5.0 MES buffer, 23.9 mg of 1-ethyl-3-[3-dimethylaminopropyl]carbodiimide hydrochloride (EDC) and 14.3 mg of N-hydroxysuccinimide (NHS) will be added. The two syringes will be connected to each other through a connector and be mixed 20 times by repetitive insertion/retraction of the syringe plungers. The resulting mixture will be transferred to a vial and allowed to stand for about 6 hours before being further purified by dialysis against PBS using a dialysis tube with a molecular weight cutoff of 20,000 Daltons. The final solution will be lyophilized to a dry powder. The resulting non-crosslinked HA (HA-HMDA) will have primary amine functional groups, i.e., the non-crosslinked HA-HMDA comprises primary ammonium groups at physiological pH.
(ii) Non-crosslinked cationic HA comprising guanidinium ions. Non-crosslinked HA-HMDA (as prepared above) will be reacted with 1*H*-Pyrazole-1-carboxamidine hydrochloride at pH 10 PBS buffer. Briefly, 10 mg of non-crosslinked HA-HMDA will be dissolved in 5 ml of pH 10 PBS buffer, then 20 mg of 1H-pyrazole-1-carboxamidine hydrochloride will be added. The reaction will be allowed to stand for about 5 hrs. The resulting solution will be dialyzed against pH 7 PBS buffer using a dialysis tube with a molecular weight cutoff of 20,000 Daltons. The final solution will be lyophilized to a dry powder. The resulting non-crosslinked cationic HA will bear guanidinium groups.
(iii) Crosslinked HA comprising primary ammonium ions. About 50 mg of crosslinked HA (provided as in Example 1B) and 14.4 mg of HMDA will be dissolved in pH 5.0 MES buffer. Then, to a separate 15 mL syringe containing 5 ml of pH 5.0 MES buffer, 23.9 mg of EDC and 14.3 mg of NHS will be added. The two syringes will be connected to each other through a connector and be mixed 20 times by repetitive insertion/retraction of the syringe plungers. The resulting mixture will be transferred to a vial and allowed to stand for about 6 hours before being further purified by dialysis using a dialysis tube with molecular weight cutoff of 20,000 Daltons. The final solution will be lyophilized to dry powder. The resulting crosslinked HA-HMDA will have primary amine functional groups, i.e., the crosslinked HA-HMDA will bear primary ammonium groups at physiological pH.
(iv) Crosslinked HA comprising guanidinium ions. Crosslinked HA-HMDA (as prepared above) will be reacted with 1*H*-Pyrazole-1-carboxamidine hydrochloride at pH 10 PBS buffer. Briefly, 10 mg of crosslinked HA-HMDA will be dissolved in 5 ml of pH 10 PBS buffer, then 20 mg of 1H-pyrazole-1-carboxamidine hydrochloride will be added. The reaction will be allowed to stand for about 5 hrs. The resulting solution will be dialyzed against pH 7 PBS buffer. The final solution will be lyophilized to dry powder. The resulting crosslinked cationic HA will bear guanidinium groups.

### D. Modified Anionic HA.

(i) Non-crosslinked HA modified with phosphonic acid functional groups. The material will be prepared by reacting non-crosslinked HA with aminophosphonic acid using EDC chemistry. Briefly, in a 15 ml syringe, about 50 mg of non-crosslinked HA (MW: 300~800 dalton) and 27.8 mg of aminomethlyphosphonic acid will be dissolved in pH 5.0 MES buffer. Then, to a separate 15 mL syringe containing 5 ml of pH 5.0 MES buffer, 23.9 mg EDC and 14.3 mg of NHS will be added. The two syringes will be connected to each other through a connector and be mixed about 20 times by repetitive insertion/retraction of the syringe plungers. The resulting mixture will be transferred to a vial and allowed to stand for about 6 hours before being further purified by dialysis using a dialysis tube with molecular weight cutoff of 20,000 Daltons. The final solution will be lyophilized to dry powder to provide non-crosslinked anionic HA comprising phosphonate groups.
(ii) Non-crosslinked HA modified with sulfonic acid functional groups. The material will be prepared using a similar procedure as described in Example 1D(i), with aminosulfamic acid used in place of aminophosphonic acid to functionalize the HA, providing non-crosslinked anionic HA comprising sulfonate groups.
(iii) Crosslinked HA modified with phosphonic acid functional groups. Crosslinked HA (provided as in Example B) is modified with phosphonic acid functional groups under conditions essentially as described in Example 1D(i), to provide crosslinked HA comprising phosphonic acid groups. Alternatively, non-crosslinked HA modified with phosphonic acid groups, as described in Example D(i), is crosslinked using HA crosslinking procedures as described by Lebreton (US 2010/0226988, US 2008/0089918, US 2010/0028438 and US 2006/0194758; *supra*), and Njikang et al. (*supra*), to provide crosslinked HA comprising phosphonic acid groups.
(iv) Crosslinked HA modified with sulfonic acid functional groups. Crosslinked HA (provided as in Example 1B) is modified with sulfonic acid functional groups under the conditions essentially as described in Example 1D(ii), to provide crosslinked HA comprising sulfonic acid groups. Alternatively, non-crosslinked HA modified with sulfonic acid groups, as described in Example D(ii), is crosslinked using HA crosslinking procedures as described by Lebreton (US 2010/0226988, US 2008/0089918, US 2010/0028438 and US 2006/0194758; *supra*), and Njikang et al. (*supra*), to provide crosslinked HA comprising sulfonic acid groups.

### E. Chitosans.

(i) Highly purity chitosan (HPC) with M_{w} 60,000-120,000 was purchased from Sigma-Aldrich. HPC with molecular weight of about 10,000 Da to about 1,000,000 Da is obtained from commercial sources.
(ii) Trimethyl chitosan is available from commercial sources, or may be prepared by established methods of alkylating chitosan (such as HPC).

### Example 2. Preparation of hydrogel using non-crosslinked HA and cationic HA.

In a 10 ml syringe, about 25 mg of non-crosslinked HA with molecular weight of about 1 million to about 3 million Daltons is hydrated with 5 ml of 1x PBS. The non-crosslinked cationic HA (Example 1C) is separately dissolved in 1 x PBS at a concentration of about 1 wt%. The hydrated HA is mixed with the non-crosslinked cationic HA at a variable charge ratio (-COOH/guanidinium) of 1:20 to 20:1 until a coacervate gel is achieved. Additional centrifigation may be applied to remove excessive water.

### Example 3. Preparation of hydrogel using crosslinked HA of high G' and cationic HA.

In a 10 ml syringe, about 2 ml of crosslinked HA with a storage modulus (G') of about 500 Pa to about 1000 Pa is mixed with 1 wt% non-crosslinked cationic HA containing guanidinium cationic groups (prepared as described in Example 1C(ii)) in pH 7.5 PBS buffer at a variable charge ratio (-COOH/guanidinium) of 1:10 to 50:1 until a coacervate gel is achieved.

### Example 4. Preparation of hydrogel using Juvederm® Ultra Plus and chitosan

*Chitosan solution preparation:* Highly pure chitosan (HPC; see Example 1E; M_{w} 85 kDa, degree of acetylation 24.4 MOL %) was dissolved in acidic water (4.904 mL milliQ water with 96 microliters concentrated HCI) to a final concentration of 38.76 mg/mL, after which the solution was steam sterilized (128°C, 5 min). The resulting HPC solution was then further diluted to various concentrations ranging from 0.4 mg/ml to 1.9 mg/ml using milliQ water, and the diluted solutions were used to prepare the different hydrogel formulations.

*Hydrogel Preparation:* Juvederm® Ultra Plus (JUP; 1 cc) was mixed with 50 microliters of each of the diluted HPC aqueous solution (0.4 mg/ml to 1.9 mg/ml) to yield gel mixtures with the equivalent ratio of HPC to JUP ranging from 0.04:1 to 0.2:1. The gels were subjected to rheology tests and swell/dissociation tests.

*Mechanical properties of the hydrogel formulations:* Following mixing, changes in the physical appearance and moduli of the hydrogels were observed (**Figure 3**). With progressively increasing concentrations of HPC, the hydrogel material appeared more opaque.

*Determination of gel rheological properties.* An oscillatory parallel plate rheometer (Anton Paar, Physica MCR 302) was used to measure the rheological properties of the gels. The diameter of the plate used was 25 mm. The gap between the plates was set at 1 mm. For each measurement, a frequency sweep at a constant strain was run first, before the strain sweep at a fixed frequency. The G' (storage modulus) of the hydrogels was obtained from the strain sweep curve at 1% strain and a strain rate of 5 Hz. The storage modulus (G') was reduced in a stepwise manner with an increase in HPC concentration (**Figure 3B**). Over the range tested, the modulus decreased approximately 9.5% for the highest concentration of chitosan.

*Gel swell*/*dissociation test:* Each hydrogel (approximately 250 µL) was injected in a cylindrical mold and centrifuged to remove bubbles. The hydrogels were then transferred into PBS (20 mL) and incubated on an orbital shaker at 37°C and 200 RPM. Within the first 24 hours, the hydrogels reached swelling equilibrium and then retained integrity without further dissociation or scattering in PBS. While the gel without added chitosan dissociated and scattered in the PBS buffer within two days (data not shown), all formulations containing chitosan remained stable for at least 29 days **(****Figure 4****).**

### Example 5. Gel cohesive test under diluted conditions.

The hydrogels made in Example 4 were soaked in abundant PBS solution. The mixture was subjected to a shaking condition of 200 rpm. Retention of gel integrity was determined by visual observation.

### Example 6. Gel moldability test

The hydrogels made in the Examples above are evaluated for gel lifting capacity and moldability.

For a quantitative analysis of gel lifting capacity, the linear compression test is performed to evaluate the lifting capacity using a parallel plate, oscillatory rheometer (Anton Paar, MCR302). In this test, a known mass of filler is placed between the plates and the upper plate is lowered at a constant speed while measuring the normal force (opposing deformation); a higher value of the normal force represents a higher resistance to deformation.

Gel moldability is evaluated by measuring the susceptibility of an injected filler to be deformed when subjected to a defined external force. The applied force, 319 grams, is chosen to simulate the force of an injector's thumb used for manipulation of the filler. The moldability of the filler will be measured over a period of nine days in a Sprague Dawley rat model using Canfield 3D Imaging.

The gels are determined to have good lifting capacity and moldability.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

## Claims

1. A dermal filler comprising a hydrogel, wherein the hydrogel comprises:
(a) an anionic hyaluronic acid (HA); and
(b) a cationic polysaccharide,
wherein the hydrogel is a coacervate hydrogel.

2. The dermal filler of claim 1, wherein the hydrogel comprises an ionic complex between the anionic HA and the cationic polysaccharide.

3. The dermal filler of claim 1 or claim 2, wherein the anionic HA is selected from non-crosslinked HA, crosslinked HA, and a mixture thereof.

4. The dermal filler of any preceding claim, wherein the cationic polysaccharide is non-crosslinked or wherein the cationic polysaccharide is selected from a cationic HA, non-crosslinked chitosan and non-crosslinked trimethylchitosan, wherein preferably the cationic polysaccharide is non-crosslinked chitosan.

5. The dermal filler of claim 1, comprising a ratio of molar equivalents of the anionic HA to the cationic polysaccharide of about 1: 0.01 to about 1:1; preferably, about 1: 0.04 to about 1: 0.20.

6. The dermal filler of any preceding claim, further comprising (i) a cosmetic agent, or (ii) an agent selected from antioxidant, an anti-itching agent, an anti-cellulite agent, an anti-scarring agent, an anesthetic agent, an anti-irritant agent, a desquamating agent, a tensioning agent, an anti-acne agent, a skin-lightening agent, a pigmentation agent, an anti-pigmentation agent, a moisturizing agent, a vitamin, and any combination of one or more of the foregoing, wherein preferably the agent is released into the soft tissue surrounding the site of administration for at least about 3 weeks after administering the dermal filler to the soft tissue.

7. The dermal filler of any preceding claim, further comprising a physiologically acceptable carrier, wherein preferably the carrier is phosphate buffered saline or non-crosslinked HA.

8. The dermal filler of any preceding claim, wherein the hydrogel has a storage modulus (G') of from about 50 Pa to about 500 Pa.

9. The dermal filler of any preceding claim which is injectable through a needle, wherein the needle gauge is at least 27 gauge, and preferably is injectable through the needle without sizing or homogenizing the dermal filler prior to the injecting.

10. A non-therapeutic method of treating a soft tissue of a subject, the method comprising injecting a dermal filler according to any preceding claim into the soft tissue of the subject.

11. The method of claim 10, wherein the soft tissue is skin, the method comprising injecting the dermal filler into a dermal region of the subject.

12. The method of claim 10 or claim 11, wherein the treating comprises augmenting the soft tissue, improving the quality of the soft tissue, or reducing a defect of the soft tissue of the subject, wherein the defect may be a wrinkle, a scar, or a loss of dermal tissue.

13. The method of any of claims 10-12, wherein the treating comprises shaping, filling, volumizing or sculpting the soft tissue of the subject.

14. A dermal filler according to any of claims 1-9 for use in a method of treating a soft tissue of a subject, the treating comprising improving dermal homeostasis, improving skin thickness, healing a wound, or reducing a scar of the subject, and the method comprising injecting the dermal filler into the soft tissue of the subject.

15. The method of any of claims 10-13 or the dermal filler for use according to claim 14, wherein the dermal filler persists in the soft tissue of the subject for at least about: 3 months, 4 months, 5 months, or 6 months after injecting the dermal filler into the soft tissue of the subject.

## Patentansprüche

1. Dermaler Füllstoff, umfassend ein Hydrogel, wobei das Hydrogel umfasst:
(a) eine anionische Hyaluronsäure (HA) und
(b) ein kationisches Polysaccharid,
wobei das Hydrogel ein Koazervat-Hydrogel ist.

2. Dermaler Füllstoff gemäß Anspruch 1, wobei das Hydrogel einen ionischen Komplex zwischen der anionischen HA und dem kationischen Polysaccharid umfasst.

3. Dermaler Füllstoff gemäß Anspruch 1 oder Anspruch 2, wobei die anionische HA aus unvernetzter HA, vernetzter HA und einer Mischung davon ausgewählt ist.

4. Dermaler Füllstoff gemäß einem vorhergehenden Anspruch, wobei das kationische Polysaccharid unvernetzt ist oder wobei das kationische Polysaccharid aus kationischer HA, unvernetztem Chitosan und unvernetztem Trimethylchitosan ausgewählt ist, wobei das kationische Polysaccharid vorzugsweise unvernetztes Chitosan ist.

5. Dermaler Füllstoff gemäß Anspruch 1, umfassend ein Moläquivalentverhältnis von anionischen HA zu kationischem Polysaccharid von etwa 1:0,01 bis etwa 1:1, vorzugsweise etwa 1,04 bis etwa 1:0,20.

6. Dermaler Füllstoff gemäß einem vorhergehenden Anspruch, ferner umfassend (i) ein kosmetisches Mittel oder (ii) ein Mittel, ausgewählt aus einem Antioxidans, einem juckreizhemmenden Mittel, einem Anti-Cellulite-Mittel, einem Mittel gegen Narbenbildung, einem Anästhetikum, einem reizhemmenden Mittel, einem abschuppenden Mittel, einem Spannungsmittel, einem Anti-Aknemittel, einem hautaufhellenden Mittel, einem Pigmentierungsmittel, einem Anti-Pigmentierungsmittel, einem feuchtigkeitsspendenden Mittel, einem Vitamin und einer Kombination aus einem oder mehreren der Vorstehenden, wobei das Mittel vorzugsweise im Weichgewebe, das die Verabreichungsstelle umgibt, für mindestens etwa 3 Wochen nach Verabreichung des dermalen Füllstoffs an das Weichgewebe freigesetzt wird.

7. Dermaler Füllstoff gemäß einem vorhergehenden Anspruch, ferner umfassend einen physiologisch annehmbaren Träger, wobei der Träger vorzugsweise eine phosphatgepufferte Kochsalzlösung oder unvernetzte HA ist.

8. Dermaler Füllstoff gemäß einem vorhergehenden Anspruch, wobei das Hydrogel einen Speichermodul (G') von etwa 50 Pa bis etwa 500 Pa aufweist.

9. Dermaler Füllstoff gemäß einem vorhergehenden Anspruch, der mittels einer Nadel injizierbar ist, wobei die Nadelgröße mindestens die Größe 27 ist, und vorzugsweise mittels der Nadel injizierbar ist, ohne den dermalen Füllstoff vor dem Injizieren zu klassieren oder zu homogenisieren.

10. Nicht-therapeutisches Verfahren zur Behandlung eines Weichgewebes eines Subjekts, wobei das Verfahren das Injizieren eines dermalen Füllstoffs gemäß einem vorhergehenden Anspruch in das Weichgewebe des Subjekts umfasst.

11. Verfahren gemäß Anspruch 10, wobei das Weichgewebe Haut ist, wobei das Verfahren das Injizieren des dermalen Füllstoffs in eine dermale Region des Subjekts umfasst.

12. Verfahren gemäß Anspruch 10 oder Anspruch 11, wobei die Behandlung die Augmentation des Weichgewebes, die Verbesserung der Qualität des Weichgewebes oder die Reduzierung eines Defekts des Weichgewebes des Subjekts umfasst, wobei der Defekt eine Falte, eine Narbe oder ein Verlust an dermalem Gewebe sein kann.

13. Verfahren gemäß einem der Ansprüche 10-12, wobei die Behandlung das Formen, Füllen, Volumisieren oder Modellieren des Weichgewebes des Subjekts umfasst.

14. Dermaler Füllstoff gemäß einem der Ansprüche 1-9 zur Verwendung in einem Verfahren zur Behandlung eines Weichgewebes eines Subjekts, wobei die Behandlung die Verbesserung von dermaler Homöostase, Verbesserung der Hautdicke, Heilung einer Wunde oder Reduzierung einer Narbe des Subjekts umfasst und wobei die Behandlung das Injizieren des dermalen Füllstoffs in das Weichgewebe des Subjekts umfasst.

15. Verfahren gemäß einem der Ansprüche 10-13 oder dermaler Füllstoff zur Verwendung gemäß Anspruch 14, wobei der dermale Füllstoff in dem Weichgewebe des Subjekts für mindestens etwa 3 Monate, 4 Monate, 5 Monate oder 6 Monate nach Injizierung des dermalen Füllstoffs in das Weichgewebe des Subjekts bestehen bleibt.

## Revendications

1. Charge dermique comprenant un hydrogel, dans laquelle l'hydrogel comprend :
(a) un acide hyaluronique (HA) anionique ; et
(b) un polysaccharide cationique,
dans laquelle l'hydrogel est un hydrogel de coacervat.

2. Charge dermique selon la revendication 1, dans laquelle l'hydrogel comprend un complexe ionique entre le HA anionique et le polysaccharide cationique.

3. Charge dermique selon la revendication 1 ou la revendication 2, dans laquelle le HA anionique est sélectionné parmi un HA non réticulé, un HA réticulé et un mélange de ceux-ci.

4. Charge dermique selon l'une quelconque des revendications précédentes, dans laquelle le polysaccharide cationique est non réticulé ou dans laquelle le polysaccharide cationique est sélectionné parmi un HA cationique, du chitosane non réticulé et du triméthylchitosane non réticulé, dans laquelle de préférence le polysaccharide cationique est du chitosane non réticulé.

5. Charge dermique selon la revendication 1, comprenant un rapport d'équivalents molaires du HA anionique sur le polysaccharide cationique d'environ 1:0,01 à environ 1:1 ; de préférence, d'environ 1:0,04 à environ 1:0,20.

6. Charge dermique selon l'une quelconque des revendications précédentes, comprenant en outre (i) un agent cosmétique, ou (ii) un agent sélectionné parmi un antioxydant, un agent anti-démangeaisons, un agent anticellulite, un agent anti-cicatrices, un agent anesthésique, un agent anti-irritant, un agent desquamant, un agent tenseur, un agent antiacnéique, un agent éclaircissant pour la peau, un agent de pigmentation, un agent antipigmentation, un agent hydratant, une vitamine et une quelconque combinaison d'un ou de plusieurs de ce qui précède, dans laquelle de préférence l'agent est libéré dans le tissu mou entourant le site d'administration pendant au moins environ 3 semaines après l'administration de la charge dermique au tissu mou.

7. Charge dermique selon l'une quelconque des revendications précédentes, comprenant en outre un support physiologiquement acceptable, dans laquelle de préférence le support est une solution saline tamponnée au phosphate ou un HA non réticulé.

8. Charge dermique selon l'une quelconque des revendications précédentes, dans laquelle l'hydrogel présente un module de stockage (G') d'environ 50 Pa à environ 500 Pa.

9. Charge dermique selon l'une quelconque des revendications précédentes, qui est injectable à travers une aiguille, dans laquelle le calibre d'aiguille est au moins calibre 27, et est de préférence injectable à travers l'aiguille sans dimensionnement ou homogénéisation de la charge dermique avant l'injection.

10. Procédé non thérapeutique de traitement d'un tissu mou d'un sujet, le procédé comprenant l'injection d'une charge dermique selon l'une quelconque des revendications précédentes dans le tissu mou du sujet.

11. Procédé selon la revendication 10, dans lequel le tissu mou est de la peau, le procédé comprenant l'injection de la charge dermique dans une région dermique du sujet.

12. Procédé selon la revendication 10 ou la revendication 11, dans lequel le traitement comprend l'augmentation du tissu mou, l'amélioration de la qualité du tissu mou ou la réduction d'un défaut du tissu mou du sujet, dans lequel le défaut peut être une ride, une cicatrice ou une perte de tissu dermique.

13. Procédé selon l'une quelconque des revendications 10-12, dans lequel le traitement comprend la mise en forme, le remplissage, la volumisation ou la sculpture du tissu mou du sujet.

14. Charge dermique selon l'une quelconque des revendications 1-9 destinée à être utilisée dans un procédé de traitement d'un tissu mou d'un sujet, le traitement comprenant l'amélioration de l'homéostasie dermique, l'amélioration de l'épaisseur de la peau, la guérison d'une plaie ou la réduction d'une cicatrice du sujet, et le procédé comprenant l'injection de la charge dermique dans le tissu mou du sujet.

15. Procédé selon l'une quelconque des revendications 10-13 ou charge dermique destinée à être utilisée selon la revendication 14, dans lequel/laquelle la charge dermique persiste dans le tissu mou du sujet pendant au moins environ : 3 mois, 4 mois, 5 mois ou 6 mois après l'injection de la charge dermique dans le tissu mou du sujet.
